# EUROPEAN PATENT SPECIFICATION

(11) **EP 4 476 353 B1**
(45) Date of publication and mention of the grant of the patent: **06.05.2026**
(21) Application number: 23716773.9
(22) Date of filing: 24.03.2023
(51) Int. Cl.: C12N 15/86, A61P 35/00, C12N 15/11, A61K 38/46

(54) **METHOD FOR THE MANUFACTURE OF A VIRAL SYSTEM, A VECTOR SYSTEM OR ANY TRANSPORT SYSTEM FOR CANCER-SPECIFIC CRISPR COMPLEXES**
VERFAHREN ZUR HERSTELLUNG EINES VIRALEN, EINES VEKTORIELLEN ODER EINES BELIEBIGEN TRANSPORTSYSTEMS UND KREBSSPEZIFISCHER CRISPR-KOMPLEXE
PROCÉDÉ DE FABRICATION D'UN SYSTÈME DE TRANSPORT VIRAL, D'UN VECTEUR OU DE TOUT SYSTÈME DE TRANSPORT ET COMPLEXES CRISPR SPÉCIFIQUES AU CANCER

(30) Priority: 01.04.2022 EP 22166327
(43) Date of publication of application: 18.12.2024
(73) Proprietor: GenCC GmbH & Co. KG, 69124 Heidelberg (DE)
(72) Inventor: WILKENS, Bodo, 64285 Darmstadt (DE); PATNAIK, Sarita, 69124 Heidelberg (DE); HESHMATPOUR, Najmeh, 68723 Oftersheim (DE); MACARRÓN PALACIOS, Arturo, 64625 Bensheim (DE); KORUS, Patrick, 64289 Darmstadt (DE)
(74) Representative: Kröncke, Rolf
(86) International application number: PCT/EP2023/057643
(87) International publication number: WO 2023/186733

(56) References cited:
- WO-A1-2017/074788
- WO-A2-2018/175502
- KWON T ET AL: "Precision targeting tumor cells using cancer-specific InDel mutations with CRISPR-Cas9", PROCEEDINGS OF THE NATIONAL ACADEMY OF SCIENCES, vol. 119, no. 9, 25 February 2022 (2022-02-25), XP055931826, ISSN: 0027-8424, DOI: 10.1073/pnas.2103532119
- KOO T ET AL: "Selective disruption of an oncogenic mutant allele by CRISPR/Cas9 induces efficient tumor regression", NUCLEIC ACIDS RESEARCH, vol. 45, no. 13, 31 May 2017 (2017-05-31), GB, pages 7897 - 7908, XP055639539, ISSN: 0305-1048, DOI: 10.1093/nar/gkx490
- REDDY A ET AL: "Genetic and Functional Drivers of Diffuse Large B Cell Lymphoma", CELL, vol. 171, no. 2, 5 October 2017 (2017-10-05), pages 481, XP085207526, ISSN: 0092-8674, DOI: 10.1016/J.CELL.2017.09.027

## Description

### BACKGROUND

The present invention relates to a method for the manufacture of individualized CRISPR/Cas complexes according to the claims comprising the steps a) identifying in a tumor specimen of a human cancer patient at least one of the following cancer specific mutations: mutation at position 127736999 on chromosome 8, mutation at position 37020625 on chromosome 9 and mutation at position 36840626 on chromosome 9; b) preparing for the at least one mutation identified in a), preferably for two or all mutations identified in a), an individualized CRISPR/Cas complex, wherein each individualized CRISPR/Cas complex comprises a guide RNA and a Cas endonuclease, wherein the guide RNA for the individualized CRISPR/Cas complex for the mutation at position 127736999 on chromosome 8 comprises a crRNA according to SEQ ID NO 37 and a tracrRNA, wherein the guide RNA for the individualized CRISPR/Cas complex for the mutation at position 37020625 on chromosome 9 comprises a crRNA according to SEQ ID NO 38 and a tracrRNA, and wherein the guide RNA for the individualized CRISPR/Cas complex for the mutation at position 36840626 on chromosome 9 comprises a crRNA according to SEQ ID NO 39 and a tracrRNA; an *in-vitro* method, for inducing cell death in cancerous or pre-cancerous cells of B-cell-lymphocytes, a composition related thereto and individualized CRISPR/Cas complexes preferably for use in the treatment of B-cell-lymphoma or for use in inducing cell death in cancerous or pre-cancerous B-cell-lymphocytes.

According to the World Health Organization (WHO), cancer represents nowadays the second leading cause of death globally. Despite improving comprehensive understanding of the pathomechanisms and the rapid advances in treatment over the last decades, cancer is with roughly 19.3 million new cancer cases and almost 10.0 million cancer-related deaths in 2020, the second leading cause of mortality and morbidity globally after cardiovascular diseases (Sung et al., 2021). Considering the rising world population and the annual cost of cancer care, cancer represents an evident health burden.

Cancer is a highly heterogeneous disease generally based on a complex process of progressive accumulation of genetic and molecular changes that result in abnormal growth of cells. During tumorigenesis, healthy cells successively acquire hallmark features including resistance to cell death, sustained proliferative signaling, evasion of growth suppressors, replicative immortality, induction of angiogenesis, and activation of invasion and metastasis (Hanahan & Weinberg, 2011).

Depending on their origin, most cancer types can be broadly classified in mesenchymal, epithelial, neuroectodermal, and hematopoietic. Hematological neoplasms may be further divided into lymphomas, leukemias, and myelomas, whereupon plenty further subtypes differing in cellular lineage, characteristics, response rate to therapy, and prognosis have been identified (Weinberg RA, 2014). Within the plethora of genetic alterations known to be associated with hematological malignancies, few aberrations in particular genomic regions have been identified to be recurrent among multiple cancer subtypes.

B cell lymphomas, a major subclass of lymphoma, represent around 5% of all newly diagnosed malignancies. This disease comprises a large variety of subtypes, including mantle cell lymphoma, Burkitt's lymphoma, diffuse large B cell lymphoma (DLBCL), follicular lymphoma, or multiple myeloma among others, depending on the stage of development of the B cell.

Current standard therapies against lymphomas and several other cancers comprise a variety of available treatments such as surgery, chemotherapy, radiation therapy, drug treatment therapy or immunotherapy among others. In general, remarkable advancements have been made during the last decades in the management of hematological malignancies. In spite of varying effectivity depending on the cancer subtype, large responsiveness has been observed for several lymphomas and leukemias upon combination of bone marrow transplantation and targeted chemotherapy. Notwithstanding initially high response rates, many patients treated with conventional strategies suffer disease relapse upon treatment interruption. This may be due to incomplete cancer remission, whereat remaining malignant cells proliferate after time, thus leading to relapse, resistance and poor prognosis (Shankland et al., 2012). In addition, conventional therapies are often accompanied by severe side effects and physical pain derived from induction of high levels of cytotoxicity in healthy, mostly rapidly proliferating cells such as cells of the digestive tract, hair follicles, and bone marrow.

Alternative therapies to chemotherapy such as targeted immunotherapy have rapidly expanded since the development of the hybridoma technology in 1975. Immunotherapy takes advantage of specific cellular receptors that are overexpressed on the surface of cancer cells. Antibodies significantly enhance the specificity of cancer therapy through the selective targeting of tumor-associated antigens. Rituximab, targeting the B cell-related receptor CD20, or tafasitamab, directed against CD19, are prominent examples of FDA-approved monoclonal antibodies successfully used for the treatment of hematological neoplasms. Great efforts have been made to optimize the functionality of monoclonal antibodies, resulting in the development of a plethora of antibody constructs with different architectures, specificities, and valences. Further, the anti-tumor activity of antibodies has been enhanced through conjugation with potent effector molecules, resulting in antibody-drug conjugates. High response rates have been achieved, for instance, with brentuximab-vedotin (BV) for the treatment of relapsed or refractory Hodgkin lymphoma patients (Makita et al., 2020). On the downside, these so-called tumor antigen markers targeted by immunotherapy are usually not exclusively present on malignant cells, but also normal cells, although in a significantly lower density. Hence, therapies directed against cellular markers often lead to adverse side effects. These therapies have several potential disadvantages resulting from the opsonisation of large immune cell subsets expressing the same tumor-associated antigen. The resulting suppression of the immune system leads to a significantly increased risk of infection and viral reactivation. In some cases, B cell lymphomas continue to develop resulting in the mutation or even shut down of the surface expression of antigen markers. Consequently, cancer cells cannot be targeted anymore by standard immunotherapies and patients frequently relapse and become insensitive to treatment.

Additional approaches in the field of immunotherapy including implementation of immune checkpoint inhibitors are being currently investigated in clinical studies (Halwani et al., 2021). Apart from immunotherapy, alternate drug therapies such as proteasome inhibitors are rapidly emerging. For example, Bortezomid (Velcade^{®}), carfilzomib (Kyprolis^{®}), and ixazomib (Ninlaro^{®}) have been FDA-approved for various hematological indications (Fricker, 2020). Moreover, proteolysis-targeting chimeras (PROTACs) have arisen as a revolutionary class of drug (Sun et al., 2018). Further, the revolutionary conception of using RNA for the development of individualized cancer vaccines (Sahin U et al., 2012) (WO 2012/159754) and adoptive T cell therapy have opened new avenues. Axicabtagene (KTA-CD19, Yescarta^{®}) and tisagenlecleucel (Kymriah^{®}) are two chimeric antigen receptor (CAR) T cell technologies FDA-approved for the treatment of various lymphoma diseases. Such recent treatment options, however, often include an associated high risk of cytokine release syndrome (CRS) and severe cardiotoxic effects (Sheikh et al., 2021).

Overall, despite high initial response rates and promising results in frontline and new therapeutic options, the inherent difficulties of producing patient-tailored treatment strategies have impeded further progress. Often less than 25% of treated patients are benefited from the existing generalized therapies. Many patients experience severe cytotoxicity of healthy cells and imbalances in immune responses. Furthermore, these therapies are often challenged due to tumor heterogeneity, while malignant cells are often able to resist drug treatment, leading to poor response rates and unfavorable prognosis. There is a critical need for alternative and more personalized, i.e. individualized, strategies that target genomic tumor mutations, thereby selectively eliminating cancer cells while protecting healthy body cells.

Engineered nucleases, such as transcription activator-like effector nucleases (TALENs), zinc finger nucleases (ZFNs), and mega ribozymes, have remarkably raised the evolution of gene editing and genome engineering for precision medicine. Recently, the clustered regularly interspaced short palindromic repeats (CRISPR)-associated protein Cas 9 (Cas9) has further intensified this progress, enabling the reliable and efficient modification, replacement, and insertion of DNA sequences. In bacteria and archaea, the CRISPR-Cas system identifies and processes foreign DNA or RNA fragments into CRISPR-derived RNA (crRNA), thus mounting an immune response against viral infection. Upon repeated invasion, the Cas endonuclease induces double-strand breaks (DSBs) contiguous to the protospacer adjacent motif (PAM), eradicating invading microorganisms. In the host cell, DSBs induced by the CRISPR-Cas system are repaired via non-homologues end joining (NHEJ) and homology directed repair (HDR). The error-prone mechanism of NHEJ often causes base insertions and deletions (indels), leading to frameshift mutations and premature stop codons and eventually gene loss-of function. By contrast, the rather precise mechanism of HDR applies a donor DNA template for DSB repair. Yet, both repair machineries can result in gene knockout, loss-of-function, or knockin (Wu & Cao, 2019).

Six different types and 33 subtypes of CRISPR showing different properties and CRISPR-Cas loci have been identified to date (Xing & Meng, 2020). Engineering of the CRISPR-Cas system has allowed for a wide variety of genomic applications. Moreover, multiple strategies for CRISPR-based high-throughput screening have been developed for the identification of novel therapeutic targets or biomarkers (Ahmad & Amiji, 2018). For instance, pooled CRISPR libraries have been established and used for genome-wide identification of tumor-driving genes as well as genes involved in survival signaling pathways or drug resistance. These defined genes can be subsequently used as drug targets to impair tumor cell growth (Gallipoli et al., 2018). Hence, a large list of potential genes has been proposed as therapeutic targets for a plethora of cancer cell lines. Alternatively, the feasibility of employing the CRISPR-Cas technology to correct genomic alterations, (e.g. point mutations) for the treatment of hereditary diseases has been successful (Chen et al., 2021). The correction of individual genomic changes may be, however, extremely challenging for cancer therapy considering the large heterogeneity present in most tumors.

To recapitulate, these platforms aim at the determination of tumor-driving genes for the development of biological drugs, the identification of genomic alterations, or the correction of particular genetic aberrations. However, no successful technology has been reported to date aiming at the direct induction of cell death via CRISPR-Cas-mediated genome editing in a patient- and cancer-specific manner.

Recently, Shuber A. P. described the use of the CRISPR-Cas system to induce cancer cell death via Cas endonucleases directed against mutated sequences obtained from a cancer cell, but not present in healthy cells of a subject (Shuber A.P, 2018). Thus, a Cas endonuclease complex may kill cancer cells harboring genomic instability, while being inert and not damaging normal cells. Shuber describes the use of CRISPR to target any sequence of a subject associated with genomic instability (i.e., gene inversion, deletion, loss of heterozygosity, and genetic rearrangement). However, the criteria for increasing specificity and target selection have not been stated. Moreover, no experimental evidence demonstrating the compositions and methods mentioned in the disclosure are provided.

Kwon T. et al, PNAS, vol. 119, no. 9, 2022, DOI: 10.1073/pnas. 2103532119 refers to precision targeting tumor cells using cancer-specific InDel mutations with CRISPR-Cas9. In WO 2017/074788 A1, compositions and methods for targeting cancer-specific sequence variations are described. Koo T. et al., NAR, vol. 45, no. 13, 31, 2017, pages 7897-7908 refers to selective disruption of an oncogenic mutant allele by CRISPR/Cas9 induces efficient tumor regression. WO 2018/175502 A2 discloses treating cancer with Cas endonuclease complexes. Reddy A. et al, CELL, vol. 171, no. 2, page 481 genetic and functional drivers of diffuse large B cell lymphoma are described.

Accordingly, there is a need for products and/or methods in view of novel cancer strategies which allow the discrimination between cancerous or pre-cancerous and healthy cells that may be considered as a beneficial cancer therapy. Thus, the problem underlying the present invention is to provide products and/or methods which allow novel strategies for cancer therapy and cancer prophylaxis based on the discrimination between cancerous or pre-cancerous and healthy cells. Another aim for the same purpose underlying the present invention is to provide products and/or methods for alternative and more appropriate strategies that target genomic tumor mutations, thereby selectively eliminating cancer cells while allowing the protection of healthy body cells.

### DESCRIPTION OF THE INVENTION

This need is met by the present invention with methods, compositions and complexes according to the present invention.

In a first aspect, the problem underlying the present invention is solved by a method for the manufacture of individualized CRISPR/Cas complexes comprising the steps a) identifying in a tumor specimen of a human cancer patient at least one of the following cancer specific mutations: mutation at position 127736999 on chromosome 8, mutation at position 37020625 on chromosome 9 and mutation at position 36840626 on chromosome 9; b) preparing for the at least one mutation identified in a), preferably for two or all mutations identified in a), an individualized CRISPR/Cas complex, wherein each individualized CRISPR/Cas complex comprises a guide RNA and a Cas endonuclease, wherein the guide RNA for the individualized CRISPR/Cas complex for the mutation at position 127736999 on chromosome 8 comprises a crRNA according to SEQ ID NO 37 and a tracrRNA, wherein the guide RNA for the individualized CRISPR/Cas complex for the mutation at position 37020625 on chromosome 9 comprises a crRNA according to SEQ ID NO 38 and a tracrRNA, and wherein the guide RNA for the individualized CRISPR/Cas complex for the mutation at position 36840626 on chromosome 9 comprises a crRNA according to SEQ ID NO 39 and a tracrRNA, wherein the tumor specimen is from B-cell-lymphoma.

The mutations as used in the method for the manufacture of individualized CRISPR/Cas complexes according to the present invention are each located on a tumor driver gene. They are located on the tumor driver gene PAX5 or MYC. These genes are relevant for cell survival and/or proliferation. Paired box protein PAX5 is a protein that in humans is encoded by the PAX5 gene. The PAX5 gene is a member of the paired box (PAX) family of transcription factors. MYC is a family of regulator genes and proto-oncogenes that code for transcription factors. The MYC family consists of three related human genes: c-myc (MYC), I-myc (MYCL), and n-myc (MYCN). c-myc (herein referred to as MYC) was the first gene to be discovered in this family. MYC is a transcription factor, an oncogene that is dysregulated, translocated and mutated in several cancers including lymphomas. It is associated with very aggressive clinical behaviour. It is an interactor of PAX5 and has a similar role in B-cell lymphoma survival. The mutations can be found in mutated PAX5 and MYC genes in cancer cells and not in wild-type PAX5 and MYC genes in healthy (i.e. non-cancerous or non-pre-cancerous) genes. The sequences of the human PAX5 gene and the human MYC gene are provided by UniProtKB - Q02548 (PAX5_HUMAN) as disclosed in SEQ ID NO 41 and UniProtKB - P01106 (MYC_HUMAN) as disclosed in SEQ ID NO 42.

In the human genome, the MYC variant (C>T) at position 127736999 is located on chromosome 8, the PAX5 variant (A>C) at position 37020625 is located on chromosome 9 and the PAX5 variant (G>A) at position 36840626 is located on chromosome 9. A variant herein means a mutation at the specific position in the respective gene in comparison to the specific position in its wild-type in a healthy (i.e. non-cancerous or non-pre-cancerous) gene. Position 127736999 on chromosome 8 is located at +3 position upstream of a protospacer adjacent motif (PAM). Position 37020625 on chromosome 9 is creating a new PAM. Position 36840626 on chromosome 9 is located at +2 position upstream of a PAM.

The PAM related to position 127736999 on chromosome 8 comprises or consists of the sequence AGG. The PAM related to position 37020625 on chromosome 9 comprises or consists of the sequence AGG. The PAM related to position 36840626 on chromosome 9 comprises or consists of the sequence GGG.

A tracrRNA according to the present invention can be each tracrRNA that is suitable. A preferred tracrRNA has the sequence GUUUUAGAGC UAUGCU according to SEQ ID NO40. This tracrRNA sequence is part of SEQ ID NOs 1 to 9 as disclosed below.

According to the present invention, cancerous cells are cells that divide continually, forming solid tumors or flooding the blood or lymph with abnormal cells. Cell division is a normal process used by the body for growth and repair. A parent cell divides to form two daughter cells, and these daughter cells are used to build new tissue or to replace cells that have died because of aging or damage. Healthy cells stop dividing when there is no longer a need for more daughter cells, but cancerous cells continue to produce copies. Precancerous cells are abnormal cells that could undergo changes and turn into cancer cells as time goes by. Pre-cancerous cells simply means there are cells that have grown abnormally, causing their size, shape or appearance to look different than normal, i.e. healthy, cells.

According to the present invention, normal cells, also cited as healthy cells herein, are cells that are not cancerous or pre-cancerous cells. Preferably, normal/healthy cells are cells that does not include the mutations as described herein on the respective chromosomes.

In a preferred embodiment, two of the mutations according to step a) are identified and two individualized CRISPR/Cas complexes according to step b) are prepared in a method for the manufacture of individualized CRISPR/Cas complexes according to the present invention.

In a preferred embodiment, all of the mutations according to step a) are identified and all individualized CRISPR/Cas complexes according to step b) are prepared in a method for the manufacture of individualized CRISPR/Cas complexes according to the present invention.

The term "individualized CRISPR/Cas complex" used herein has the meaning that this CRISPR/Cas complex is designed based on individual information, preferably on one or more mutations at positions in the sequence on a chromosome in cancerous or pre-cancerous cells compared to the wild type sequence on the chromosome in healthy, i.e. non-cancerous, cells, obtained from a human cancer patient or a patient supposed to have or to develop cancer in the near future. It has to be noted that not every human cancer patient shows one or more mutations at position 127736999 on chromosome 8, at position 37020625 on chromosome 9 and/or at position 36840626 on chromosome 9. Only those having at least one of these mutations will benefit from an administration of the individualized CRISPR/Cas complexes of the present invention, preferably manufactured acccording to a method for the manufacture of individualized CRISPR/Cas complexes of the present invention and further methods, complexes an compositions according to the present invention as disclosed herein.

In a preferred embodiment a method for the manufacture of individualized CRISPR/Cas complexes comprises the steps a) identifying in a tumor specimen of a human cancer patient the following cancer specific mutations: mutation at position 127736999 on chromosome 8 and mutation at position 37020625 on chromosome 9; b) preparing for each mutation identified in a) an individualized CRISPR/Cas complex, wherein each individualized CRISPR/Cas complex comprises a guide RNA and a Cas endonuclease, wherein the guide RNA for the individualized CRISPR/Cas complex for the mutation at position 127736999 on chromosome 8 comprises a crRNA according to SEQ ID NO 37 and a tracrRNA and wherein the guide RNA for the individualized CRISPR/Cas complex for the mutation at position 37020625 on chromosome 9 comprises a crRNA according to SEQ ID NO 38 and a tracrRNA. In a further embodiment according to the present invention the guide RNA for the individualized CRISPR/Cas complex for the mutation at position 127736999 on chromosome 8 comprising a sequence according to SEQ ID NO 4 and the guide RNA for the individualized CRISPR/Cas complex for the mutation at position 37020625 on chromosome 9 comprising a sequence according to SEQ ID NO 5 are prepared.

In a preferred embodiment a method for the manufacture of individualized CRISPR/Cas complexes comprises the steps a) identifying in a tumor specimen of a human cancer patient the following cancer specific mutations: mutation at position 36840626 on chromosome 9 and mutation at position 37020625 on chromosome 9; b) preparing for each mutation identified in a) an individualized CRISPR/Cas complex, wherein each individualized CRISPR/Cas complex comprises a guide RNA and a Cas endonuclease, wherein the guide RNA for the individualized CRISPR/Cas complex for the mutation at position 36840626 on chromosome 9 comprises a crRNA according to SEQ ID NO 39 and a tracrRNA and wherein the guide RNA for the individualized CRISPR/Cas complex for the mutation at position 37020625 on chromosome 9 comprises a crRNA according to SEQ ID NO 38 and a tracrRNA. In a further embodiment according to the present invention the guide RNA for the individualized CRISPR/Cas complex for the mutation at position 36840626 on chromosome 9 comprising a sequence according to SEQ ID NO 6 and the guide RNA for the individualized CRISPR/Cas complex for the mutation at position 37020625 on chromosome 9 comprising a sequence according to SEQ ID NO 5 are prepared.

Each guide RNA prepared according to the present invention is directed against a nucleic acid sequence that is specific for a mutation occuring in cancerous or pre-cancerous cells compared to the nucleic acid sequence at the same position occuring in healthy, i.e. non-cancerous or non-pre-cancerous, cells.

In a further preferred embodiment the tumor specimen is selected from the group comprising glioma cells, astrocytoma cells, neuroblastoma cells, meningioma cells, glioblastoma cells, urothelial carcinoma cells, bladder papilloma cells, rhabdomyosarcoma cells, osteosarcoma cells, Ewing's sarcoma cells, chondrosarcoma cells, mammary carcinoma cells, breast adenocarcinoma cells, squamous carcinoma cells, cervical adenocarcinoma cells, choriocarcinoma cells, leiomyosarcoma cells, colorectal adenocarcinoma cells, colon carcinoma cells, hepatoblastoma cells, hepatic adenocarcinoma cells, gall bladder adenocarcinoma cells, epithelial appendiceal cancer cells, neuroendocrine appendiceal cancer cells, goblet cell carcinomas, cholangiocarcinoma cells, gastrointestinal carcinoid tumor cells, pancreas adenocarcinoma cells, neuroendocrine carcinoma cells, renal adenocarcinoma cells, renal carcinoma cells, pharynx carcinoma cells, neck squamous carcinoma cells, thyroid carcinoma cells, nasal squamous carcinoma cells, oesophagus squamous cells, submaxillary gland adenoma cells, cone precursors cells, neuroendocrine cells, prostate luminal cells, prostatic adenocarcinoma cells, pancreas adenocarcinoma cells, cervix carcinoma cells, lung carcinoma cells, lung squamous cell carcinoma (SCC) cells, lung epidermoid carcinoma cells, hepatoblastoma (hepatocellular carcinoma) cells, hepatic adenocarcinoma cells, Alexander hepatoma cells, ovary adenocarcinoma cells, endometrium adenocarcinoma cells, basal carcinoma cells, skin squamous carcinoma cells

According to the present invention the tumor specimen is from B-cell-lymphocytes.

According to the present invention, "specimen" and "sample" are used interchangeable having the same meaning.

In a preferred embodiment a method for the manufacture of individualized CRISPR/Cas complexes comprises the steps a) identifying in a tumor specimen of a human cancer patient the following cancer specific mutations: mutation at position 127736999 on chromosome 8 and mutation at position 37020625 on chromosome 9; b) preparing for each mutation identified in a) an individualized CRISPR/Cas complex, wherein each individualized CRISPR/Cas complex comprises a guide RNA and a Cas endonuclease, wherein the guide RNA for the individualized CRISPR/Cas complex for the mutation at position 127736999 on chromosome 8 comprises a sequence according to SEQ ID NO 4 and wherein the guide RNA for the individualized CRISPR/Cas complex for the mutation at position 37020625 on chromosome 9 comprises a sequence according to SEQ ID NO 5, and wherein the tumor specimen is from B-cell-lymphocytes. Preferably, the Cas endonucleases according to this embodiment are Cas9 endonucleases.

In a preferred embodiment a method for the manufacture of individualized CRISPR/Cas complexes comprises the steps a) identifying in a tumor specimen of a human cancer patient the following cancer specific mutations: mutation at position 36840626 on chromosome 9 and mutation at position 37020625 on chromosome 9; b) preparing for each mutation identified in a) an individualized CRISPR/Cas complex, wherein each individualized CRISPR/Cas complex comprises a guide RNA and a Cas endonuclease, wherein the guide RNA for the individualized CRISPR/Cas complex for the mutation at position 36840626 on chromosome 9 comprises a sequence according to SEQ ID NO 6 and wherein the guide RNA for the individualized CRISPR/Cas complex for the mutation at position 37020625 on chromosome 9 comprises a sequence according to SEQ ID NO 5, and wherein the tumor specimen is from B-cell-lymphocytes. Preferably, the Cas endonucleases according to this embodiment are Cas9 endonucleases.

A Cas endonuclease according to the present invention and as used herein is a type I, type II or type III endonuclease, preferably a type I endonuclease, more preferably a CRISPR/Cas endonuclease, most preferably a CRISPR/Cas9 endonuclease. Any Cas endonuclease may be used in the disclosed methods, complexes and compositions. The guide RNA may be covalently linked to the Cas endonuclease. For example, the Cas endonuclease may be a Cas9 and cut DNA. A complex including a Cas9 endonuclease may be referred to as a CRISPR/Cas9 complex. In another example, the Cas endonuclease may be a Cas12a and cut RNA. A complex including the Cas12a endonuclease may be referred to as a CRISPR/Cas12a complex.

In a second aspect, the problem underlying the present invention is solved by an *in-vitro* method, for inducing cell death in cancerous or pre-cancerous cells of B-cell-lymphocytes, comprising the steps:
a) isolation of B-cell-lymphocytes from one or more specimen obtained from a human cancer patient or a human subject supposed to have or to develop cancer, whereby non-cancerous B-cell-lymphocytes and cancerous or pre-cancerous B-cell-lymphocytes are obtained;
b) cultivation, preferably *in-vitro* cultivation, of the B-cell-lymphocytes from the one or more specimen obtained in a);
c) identifying in the cancerous or pre-cancerous B-cell-lymphocytes at least one of the following cancer specific mutations: mutation at position 127736999 on chromosome 8, mutation at position 37020625 on chromosome 9 and mutation at position 36840626 on chromosome 9;
d) preparing an individualized CRISPR/Cas complex for each of the cancer specific mutations identified in c) according to step b) of a method for the manufacture of individualized CRISPR/Cas complexes according to the present invention and preferably as described in at least one of the embodiments as disclosed herein,
e) adding the one or more prepared individualized CRISPR/Cas complexes to the cultivated B-cell-lymphocytes obtained in step b), and
f) optionally, determining if cell death is induced in the cancerous or pre-cancerous B-cell-lymphocytes of said cultivated B-cell-lymphocytes.

According to the present invention cell death is preferably induced by gene knockout as a consequence of double strand breaks. Furthermore, according to the present invention, the induction of cell death is preferably reached through apoptosis.

In a preferred embodiment of this aspect, step f) further includes determining if cell death is not induced in the non-cancerous B-cell-lymphocytes of said cultivated B-cell-lymphocytes. It is further preferred that cell death is induced in the cancerous or pre-cancerous B-cell-lymphocytes of said cultivated B-cell-lymphocytes only.

In a preferred embodiment of an *in-vitro* method for inducing cell death in cancerous or pre-cancerous cells of B-cell-lymphocytes according to the present invention the following two mutations are identified in step b): mutation at position 127736999 on chromosome 8 and mutation at position 37020625 on chromosome 9.

In another preferred embodiment of an *in-vitro* method for inducing cell death in cancerous or pre-cancerous cells of B-cell-lymphocytes according to the present invention the following two mutations are identified in step b): mutation at position 36840626 on chromosome 9 and mutation at position 37020625 on chromosome 9.

In a third aspect, the problem underlying the present invention is solved by a composition comprising one or more individualized CRISPR/Cas complexes manufactured according to the method for the manufacture of individualized CRISPR/Cas complexes of the present invention, preferably according to at least one of the embodiments as described herein.

In a forth aspect, the problem underlying the present invention is solved by an individualized CRISPR/Cas complex comprising a guide RNA and a Cas endonuclease, preferably a Cas9 endonuclease, wherein the guide RNA comprises a crRNA according to SEQ ID NO 37 and a tracrRNA, a crRNA according to SEQ ID NO 38 and a tracrRNA, or a crRNA according to SEQ ID NO 39 and a tracrRNA.

In a preferred embodiment, the individualized CRISPR/Cas complex according to the present invention comprises a guide RNA according to SEQ ID NO 4, SEQ ID NO 5 or SEQ ID NO 6. Preferably, two individualized CRISPR/Cas complexes according to the present invention are combined. Preferably, the two individualized CRISPR/Cas complexes are individualized CRISPR/Cas complexes comprising guide RNAs according to SEQ ID NO 4 and SEQ ID NO 5 or individualized CRISPR/Cas complexes comprising guide RNAs according to SEQ ID NO 6 and SEQ ID NO 5. In one embodiment, all three individualized CRISPR/Cas complexes according to the present invention can be combined.

In a fifth aspect, the problem underlying the present invention is solved by an individualized CRISPR/Cas complex manufactured according to the method for the manufacture of individualized CRISPR/Cas complexes of the present invention, a composition comprising one or more of such individualized CRISPR/Cas complexes or at least one, preferably two or more, CRISPR/Cas complexes comprising a crRNA according to SEQ ID NO 37 and a tracrRNA, a crRNA according to SEQ ID NO 38 and a tracrRNA, or a crRNA according to SEQ ID NO 39 and a tracrRNA, preferably at least one, preferably two or more, CRISPR/Cas complexes comprising a guide RNA according to SEQ ID NO 4, SEQ ID NO 5 or SEQ ID NO 6, for use in the treatment of B-cell-lymphoma.

The inventors of the present invention have developed individualized CRISPR/Cas complexes of the present invention as described herein which have surprisingly shown that they can induce cell death in cancerous or pre-cancerous B-cell-lymphocytes only but not in healthy, i.e. non-cancerous or non-pre-cancerous, B-cell-lymphocytes. Accordingly, these individualized CRISPR/Cas complexes according to the invention are or at least seem to be suitable for the use in the treatment of B-cell-lymphoma.

In a sixth aspect, the problem underlying the present invention is solved by an individualized CRISPR/Cas complex manufactured according to the method for the manufacture of individualized CRISPR/Cas complexes of the present invention, a composition comprising one or more of such individualized CRISPR/Cas complexes or at least one, preferably two or more, CRISPR/Cas complexes comprising a crRNA according to SEQ ID NO 37 and a tracrRNA, a crRNA according to SEQ ID NO 38 and a tracrRNA, or a crRNA according to SEQ ID NO 39 and a tracrRNA, preferably at least one, preferably two or more, CRISPR/Cas complexes comprising a guide RNA according to SEQ ID NO 4, SEQ ID NO 5 or SEQ ID NO 6, for inducing cell death in cancerous or pre-cancerous B-cell-lymphocytes. According to the present invention, cell death is not induced in healthy, i.e. non-cancerous or non-pre-cancerous, B-cell-lymphocytes by the use of such one, two or more individualized CRISPR/Cas complexes or compositions comprising one, two or more of such individualized CRISPR/Cas complexes.

Further, a method for the manufacture of a transport system is described herein not being part of the claims comprising the steps
a) isolation of a class of cells from one or more sample(s) obtained from a subject;
b) identifying a nucleic acid sequence that is specific for a subgroup of the class of cells;
c) design of guide RNA(s) and/or DNA and/or protein directed against the nucleic acid sequence, and
d) cloning of one or more sequence(s) encoding the guide RNA(s) and a Cas endonuclease into a viral system or a vector system, preferably into a viral vector, or including the guide RNA(s) and a Cas endonuclease into a transport system.

The term "a transport system" as used herein refers to a viral transport system (viral system), a vector transport system (vector system), a combination thereof (viral vector system) or any other transport system which can be used in a method according to the present invention. According to the present invention a viral vector system is preferred. Specifically, a lentiviral vector system is preferred. By "transport" as used herein it is referred to the transport of information and/or the transport of material. For example, a viral transport system may be a virus or virus-like particles (VLPs) whose viral capsid shells encases RNA(s), guide RNA(s), DNA, protein, Cas endonucleases and the like. RNA(s), guide RNA(s), DNA, protein, Cas endonucleases and the like are examples of material. The virus or the VLPs are the transporter of this material. In another example, a viral vector system may include one or more sequences encoding for RNA, guide RNA, DNA, protein, Cas endonucleases and the like. Sequences encoding for RNA, guide RNA, DNA, protein, Cas endonucleases and the like are examples of information. Thereby, the viral vector system transports the information. Every transport system may be used that can either transport the material or the information to the desired place of action. A transport system is preferably a delivery system that is able to transport the guide RNA and the Cas endonuclease or the (viral) vector encoding them into cells, preferably into cells of a subject, preferably into cells of the subject from which the one or more sample(s) have been obtained.

The term "guide RNA(s)" as used herein refers to one, two, three or more guide RNAs.

The transport system manufactured according to the method described herein is preferably introduced to a group of cells, preferably to a group of cancerous or pre-cancerous cells.

The "one or more sample(s)" may have been obtained in any clinically acceptable manner, and the nucleic acids may be extracted from the sample by any suitable method. Generally, nucleic acid can be extracted from a biological sample by a variety of techniques such as those described by Maniatis, et al. (Molecular Cloning: A Laboratory Manual, Cold Spring Harbor, N.Y., pp. 280-281, 1982).

The "one or more sample(s)" may be a human tissue or bodily fluid. A tissue is a mass of connected cells and/or extracellular matrix material, e.g. skin tissue, nasal passage tissue, CNS tissue, neural tissue, eye tissue, liver tissue, kidney tissue, placental tissue, mammary gland tissue, placental tissue, gastrointestinal tissue, musculoskeletal tissue, genitourinary tissue, bone marrow, and the like, derived from, for example, a human or other mammal and includes the connecting material and the liquid material in association with the cells and/or tissues. A bodily fluid is a liquid material derived from, for example, a human or other mammal. Such body fluids include, but are not limited to, mucous, blood, plasma, serum, serum derivatives, bile, maternal blood, phlegm, saliva, sweat, amniotic fluid, mammary fluid, urine, and cerebrospinal fluid (CSF), such as lumbar or ventricular CSF. A sample may also be a fine needle aspirate or biopsied tissue. A sample also may be media containing cells or biological material. In certain embodiments, the sample includes nucleic acid molecules that are cell free circulating nucleic acid molecules.

Identifying a nucleic acid sequence that is specific for a subgroup of the class of cells according to step b) of a method for the manufacture of a transport system can be determined by many methods. Preferably, differences in sequences between cancerous/pre-cancerous cells being the subgroup of the class of cells and normal/healthy cells of this class of cells may be determined by many methods, such as sequencing. Sequencing may be by a chain-termination sequencing technique (Sanger sequencing) or by a single molecule sequencing-by-synthesis technique. In certain embodiments, a nucleic acid is obtained from a cell belonging to the normal/healthy subgroup of the class of cells of the subject and sequenced, thereby acquiring a wild-type sequence. Also, a nucleic acid is obtained from a cell belonging to the cancerous or pre-cancerous subgroup of the class of cells of the same subject and sequenced, thereby acquiring a mutated sequence. Once the two different sequences are acquired, the wild-type sequence and the mutated sequences may be compared, and thus a determination of the difference between the wild-type sequence and the mutated sequence is made. The difference between the wild-type sequence and the mutated sequence is the mutated region, more specifically the identified nucleic acid sequence that is specific for the subgroup, specifically the cancerous or pre-cancerous subgroup of the class of cells, according to step b) of a method for the manufacture of a transport system according to the present invention, to which the guide RNA, DNA or protein and the Cas endonuclease will be designed. In certain embodiments, the difference between the wild-type sequence and the mutated sequence is the result of a loss of genetic material between the first and second portions in the mutated sequence, in which the loss of genetic material results from a mutation event including a deletion, a substitution, or a rearrangement.

In a preferred embodiment, a method described herein comprises genome sequence analysis, preferably whole genome sequence analysis, whole exome sequencing and/or cancer paneling, using the sample obtained from the subject and determination whether the nucleic acid sequence is unique in the subgroup of the class of cells, i.e. cancerous or pre-cancerous cells.

In certain embodiments, sequencing may be performed by the Sanger sequencing technique. Classical Sanger sequencing involves a single-stranded DNA template, a DNA primer, a DNA polymerase, radioactively or fluorescently labeled nucleotides, and modified nucleotides that terminate DNA strand elongation. If the label is not attached to the dideoxynucleotide terminator (e.g., labeled primer), or is a monochromatic label (e.g., radioisotope), then the DNA sample is divided into four separate sequencing reactions, containing four standard deoxynucleotides (dATP, dGTP, dCTP and dTTP) and the DNA polymerase. To each reaction is added only one of the four dideoxynucleotides (ddATP, ddGTP, ddCTP, or ddTTP). These dideoxynucleotides are the chain-terminating nucleotides, lacking a 3'-OH group required for the formation of a phosphodiester bond between two nucleotides during DNA strand elongation. If each of the dideoxynucleotides carries a different label, however, (e.g., 4 different fluorescent dyes), then all the sequencing reactions can be carried out together without the need for separate reactions. Incorporation of a dideoxynucleotide into the nascent (i.e., elongating) DNA strand terminates DNA strand extension, resulting in a nested set of DNA fragments of varying length. Newly synthesized and labeled DNA fragments are denatured, and separated by size using gel electrophoresis on a denaturing polyacrylamide-urea gel capable of resolving single-base differences in chain length. If each of the four DNA synthesis reactions was labeled with the same, monochromatic label (e.g., radioisotope), then they are separated in one of four individual, adjacent lanes in the gel, in which each lane in the gel is designated according to the dideoxynucleotide used in the respective reaction, i.e., gel lanes A, T, G, C. If four different labels were utilized, then the reactions can be combined in a single lane on the gel. DNA bands are then visualized by autoradiography or fluorescence, and the DNA sequence can be directly read from the X-ray film or gel image. The terminal nucleotide base is identified according to the dideoxynucleotide that was added in the reaction resulting in that band or its corresponding direct label. The relative positions of the different bands in the gel are then used to read (from shortest to longest) the DNA sequence as indicated. The Sanger sequencing process can be automated using a DNA sequencer, such as those commercially available from PerkinElmer, Beckman Coulter, Life Technologies, and others.

In other embodiments, sequencing may be accomplished by a single-molecule sequencing by synthesis technique. Single molecule sequencing is shown for example in Lapidus et al. (U.S. patent number 7,169,560), Quake et al. (U.S. patent number 6,818,395), Harris (U.S. patent number 7,282,337), Quake et al. (U.S. patent application number 2002/0164629), and Braslavsky, et al., PNAS (USA), 100: 3960-3964 (2003). Briefly, a single-stranded nucleic acid (e.g., DNA or cDNA) is hybridized to oligonucleotides attached to a surface of a flow cell. The oligonucleotides may be covalently attached to the surface or various attachments other than covalent linking may be employed. Moreover, the attachment may be indirect, e.g., via a polymerase directly or indirectly attached to the surface. The surface may be planar or otherwise, and/or may be porous or non-porous, or any other type of surface suitable for attachment. The nucleic acid is then sequenced by imaging the polymerase-mediated addition of fluorescently-labeled nucleotides incorporated into the growing strand surface oligonucleotide, at single molecule resolution. Other single molecule sequencing techniques involve detection of pyrophosphate as it is cleaved from incorporation of a single nucleotide into a nascent strand of DNA, as is shown in Rothberg et al. (U.S. patent numbers 7,335,762, 7,264,929, 7,244,559, and 7,211,390) and Leamon et al. (U.S. patent number 7,323,305),

In other embodiments, targeted resequencing can be used. Resequencing is shown for example in Harris (U.S. patent application numbers 2008/0233575, 2009/0075252, and 2009/0197257). Briefly, a specific segment of the target is selected (for example by PCR, microarray, or MIPS) prior to sequencing. A primer designed to hybridize to this particular segment, is introduced and a primer/template duplex is formed. The primer/template duplex is exposed to a polymerase, and at least one detectably labeled nucleotide under conditions sufficient for template dependent nucleotide addition to the primer. The incorporation of the labeled nucleotide is determined, as well the identity of the nucleotide that is complementary to a nucleotide an the template at a position that is opposite the incorporated nucleotide. After the polymerization reaction, the primer may be removed from the duplex. The primer may be removed by any suitable means, for example by raising the temperature of the surface or substrate such that the duplex is melted, or by changing the Buffer conditions to destabilize the duplex, or combination thereof. Methods for melting template/primer duplexes are described, for example, in chapter 10 of Molecular Cloning, a Laboratory Manual, 3.sup.rd Edition, J. Sambrook, and D. W. Russell, Cold Spring Harbor Press (2001). After removing the primer, the template may be exposed to a second primer capable of hybridizing to the template. In one embodiment, the second primer is capable of hybridizing to the same region of the template as the first primer (also referred to herein as a first region), to form a template/primer duplex. The polymerization reaction is then repeated, thereby resequencing at least a portion of the template. If the nucleic acid from the sample is degraded or only a minimal amount of nucleic acid can be obtained from the sample, PCR can be performed on the nucleic acid in order to obtain a sufficient amount of nucleic acid for sequencing (See e.g., Mullis et al. U.S. patent number 4,683,195).

In certain embodiments next-generation sequencing (NGS) may be performed. This method requires DNA enrichment and library preparation. Subsequently, NGS technology involves clonal amplification and sequencing by synthesis (SBS) chemistry to simultaneously read through independent DNA templates for the identification of DNA bases and their incorporation into a nucleic acid chain. The order of the DNA sequence is generally ascertained via detection of fluorescent signal emitted by each nucleic acid base upon addition to the growing strand. Thus, short reads of often less than 500 bp with extensive depth of coverage are generated, allowing for fast sequencing in a high-throughput manner. This technology may be used for whole genome sequencing, whole exome sequencing or deep sequencing of specific target regions of healthy as well as malignant samples, among others. Post-sequencing analysis enables identification of genetic alterations such as single nucleotide polymorphisms (SNPs), copy number variations, translocations, insertions and deletions events, or for ascertainment of tumor subclones and subpopulations. NGS can be carried out by commercial suppliers such as Illumina by using different sequencers, depending on method and application.

Furthermore, it is disclosed that once the wild-type sequences from the normal/healthy subgroup of the class of cells and the mutant sequences from the cancerous or pre-cancerous subgroup of the class of cells are obtained, these sequences can be compared to determine the differences between the sequences. The difference of interest may be an alteration or loss of genetic material from the wild-type sequence, e.g. substitution, insertion or deletion events, that result in the mutant sequence found in the cancerous or pre-cancerous subgroup of the class of cells. One of the advantages of the present invention is that a single-nucleotide polymorphism (SNP) can be used. This provides more options to choose an appropriate guide RNA.

After determining the region of genetic material that is altered in comparison to the wild-type sequence to result in the mutant sequence, the regions of the sequences that flank the mutated region in both the wild-type and mutant sequences (i.e., sequences upstream of the mutated region and downstream of the mutated region) can be analyzed. Based on the analysis of the sequences that flank the mutated region, Cas endonucleases and guide RNA molecules can be designed to hybridize and target the identified nucleic acid sequence that is specific for the subgroup of the class of cells, preferably specific for the cancerous or pre-cancerous cells and not present in the normal and healthy cells.

In a preferred embodiment, the guide RNA(s) and/or DNA and/or protein directed against the nucleic acid sequence according to step b) of a method for the manufacture of a transport system according to the present invention, is or are designed or is or are designable using for instance the SNP-CRISPR tool and/or the custom guide RNA design, preferably the SNP-CRISPT tool and/or the Custom Alt-R^{®} CRISPR-Cas9 guide RNA tool (IDT). Furthermore, the person skilled in the art is well aware how to design a desired guide RNA, DNA or protein.

According to another preferred embodiment, the nucleic acid sequence according to b) includes one or more homozygous and/or heterozygous mutation(s) and/or is located on a tumor driver gene, preferably the tumor driver gene is selected from the group comprising PAX5, MYC, mTOR and CD79B, and/or is located on genes that are relevant for cell survival and/or proliferation. It is also preferred that the one or more homozygous and/or heterozygous mutation(s) according to b) is located within or close to a PAM or creates a new PAM. It is even more preferred that the one or more homozygous and/or heterozygous mutation(s) according to b) is located at +2 to +7 position upstream of PAM or mutation-derived PAM.

As throughout this application, any data base code used refers to the sequence available from said data base on the first priority date relating to said application. For example, the sequences of PAX5, MYC, mTOR and CD79B are sequences according to UniProtKB - Q02548 (PAX5_HUMAN) as disclosed in SEQ ID NO 41, UniProtKB - P01106 (MYC_HUMAN) as disclosed in SEQ ID NO 42, UniProtKB - P42345 (MTOR_HUMAN) as disclosed in SEQ ID NO 43 and UniProtKB - P40259 (CD79B_HUMAN) as disclosed in SEQ ID NO 44.

In a further preferred embodiment, the method for the manufacture of a transport system comprises the additional steps
e) *in vitro* cultivation of the class of cells from the sample obtained from the subject,
f) adding the transport system obtained in step d) to the cultivated class of cells obtained in step e), and
g) optionally determining of whether the transport system specifically induces cell death in the subgroup of the class of cells, i.e. cancerous or pre-cancerous cells.

A method is especially preferred comprising the steps
a) isolation of a class of cells from one or more sample(s) obtained from a subject;
b) identifying a nucleic acid sequence that is specific for a subgroup of the class of cells;
c) design of guide RNA(s) and/or DNA and/or protein directed against the nucleic acid sequence;
d) cloning of one or more sequence(s) encoding the guide RNA(s) and a Cas endonuclease into a viral system or a vector system, preferably into a viral vector, or including the guide RNA(s) and a Cas endonuclease into a transport system;
e) *in vitro* cultivation of the class of cells from the sample obtained from the subject;
f) adding the transport system obtained in step d) to the cultivated class of cells obtained in step e), and
g) optionally determining if cell death is induced in the subgroup of the class of cells, i.e. cancerous or pre-cancerous cells.

The "class of cells" can be selected from the group comprising B lymphocytes, urothelial cells, bladder cells, breast cells, squamous cells, cervical cells, colorectal cells, colon cells, hepatic cells, gall bladder cells, epithelial cells, neuroendocrine cells, gastrointestinal cells, pancreas cells, thyroid cells, oesophagus cells, prostate cells, lung cells, ovary cells, endometrium cells, skin cells.

The "class of cells" is most preferably B lymphocytes.

The "subgroup of the class of cells" are cancer or pre-cancerous cells. It is also preferred that the nucleic acid sequence according to step b) of the method is located only in cancer or pre-cancerous cells.

The "subgroup of the class of cells" is preferably selected from the group comprising glioma cells, astrocytoma cells, neuroblastoma cells, meningioma cells, glioblastoma cells, urothelial carcinoma cells, bladder papilloma cells, rhabdomyosarcoma cells, osteosarcoma cells, Ewing's sarcoma cells, chondrosarcoma cells, mammary carcinoma cells, breast adenocarcinoma cells, squamous carcinoma cells, cervical adenocarcinoma cells, choriocarcinoma cells, leiomyosarcoma cells, colorectal adenocarcinoma cells, colon carcinoma cells, hepatoblastoma cells, hepatic adenocarcinoma cells, gall bladder adenocarcinoma cells, epithelial appendiceal cancer cells, neuroendocrine appendiceal cancer cells, goblet cell carcinomas, cholangiocarcinoma cells, gastrointestinal carcinoid tumor cells, pancreas adenocarcinoma cells, neuroendocrine carcinoma cells, renal adenocarcinoma cells, renal carcinoma cells, pharynx carcinoma cells, neck squamous carcinoma cells, thyroid carcinoma cells, nasal squamous carcinoma cells, oesophagus squamous cells, submaxillary gland adenoma cells, cone precursors cells, neuroendocrine cells, prostate luminal cells, prostatic adenocarcinoma cells, pancreas adenocarcinoma cells, cervix carcinoma cells, lung carcinoma cells, lung squamous cell carcinoma (SCC) cells, lung epidermoid carcinoma cells, hepatoblastoma (hepatocellular carcinoma) cells, hepatic adenocarcinoma cells, Alexander hepatoma cells, ovary adenocarcinoma cells, endometrium adenocarcinoma cells, basal carcinoma cells, skin squamous carcinoma cells.

In a preferred embodiment, the "subgroup of the class of cells" is cancerous or pre-cancerous B lymphocytes.

In another preferred embodiment, preferably according to a preferred embodiment as already disclosed, the subject from which the one or more sample(s) has been used to isolate the "class of cells" is a mammal, preferably a human.

In a further preferred embodiment, the method for the manufacture of a transport system wherein in step b) of a method according to the present invention two or more nucleic acid sequences are identified wherein both or all are specific for the subgroup of the class of cells and wherein in step c) a guide RNA, DNA or protein directed against each of the nucleic acid sequences is designed and wherein in step d) one or more sequences each of them encoding one of the guide RNAs and one or more sequences each of them encoding one Cas endonuclease are cloned into the transport system or two or more guide RNAs and two or more Cas endonucleases are included into the transport system.

Another aspect described herein but not being part of the claims is a composition comprising a transport system manufactured according to the disclosed method.

A further aspect but not being part of the claims is a composition comprising a transport system manufactured or producible according to a method as described herein, preferably manufactured or producible according to one or more preferred embodiments of a method described herein, for use in the treatment of cancer, preferably for use in the treatment of cancer selected from the group comprising brain, bladder, blood, bone, breast, cervical, colorectal, gastrointestinal, endocrine, kidney, liver, lung, ovarian, pancreatic, prostate and thyroid, most preferably for use in the treatment of B-cell-lymphoma.

In a preferred composition the Cas endonuclease is delivered as a protein complexed with the guide RNA. In another preferred composition of the present invention the Cas endonuclease is delivered as a DNA that encodes the Cas endonuclease to be transcribed in cells of the subject. In a further preferred composition the Cas endonuclease is delivered as an mRNA to be translated in cells of the subject. In a further preferred embodiment, the Cas endonuclease is a Cas9 endonuclease that cuts DNA or the Cas endonuclease is a Cas12a endonuclease that cuts RNA.

In a preferred composition a guide RNA contains a targeting sequence that is complementary to the identified nucleic acid sequence that is specific for the "subgroup of the class of cells" of the method described.

In a preferred embodiment, the composition induces cell death by generating double strand breaks in the identified nucleic acid sequence that is specific for the "subgroup of the class of cells". In another embodiment, the Cas endonuclease induces cell death by incorporating a protein coding gene sequence that results in expression of a lethal protein.

In a preferred embodiment, the composition is for use in patient-specific treatment of cancer, wherein the sample is obtained from the subject to whom the transport system is later applied.

In a preferred embodiment, the composition includes a Cas endonuclease or nucleic acid encoding the Cas endonuclease and a guide RNA or a nucleic acid encoding the guide RNA that targets the Cas endonuclease to an identified nucleic acid sequence according to b) of the method for the manufacture of a transport system of the present invention. The guide RNA may contain a targeting sequence that is complementary to the identified nucleic acid sequence according to b) of the method for the manufacture of a transport system described herein.

In a preferred embodiment, the herein disclosed compositions may be administered using any amount and any route of administration effective for treating a disease, preferably for treating cancer. In a further preferred embodiment, such compositions may be administered in an amount effective for treating a cancer. Thus, the expression "amount effective for treating a cancer", as used herein, refers to a sufficient amount of composition to beneficially prevent or ameliorate the symptoms of the cancer.

The exact dosage for compositions to be used for cancer therapy may be chosen by an individual physician in view of the subject to be treated and certain other factors. Dosage and administration are adjusted to provide sufficient levels of the composition or to maintain the desired effect. Additional factors which may be taken into account include the severity of the disease state, age, weight and gender of the subject; diet, time and frequency of administration; route of administration; drug combinations; reaction sensitivities; and tolerance/response to therapy. Long acting pharmaceutical compositions might be administered, for example, hourly, twice hourly, every 3 to four hours, daily, twice daily, every 3 to 4 days, every weck, or once every two weeks depending on half-life and clearance rate of the particular composition. The disclosed compositions may preferably be formulated in dosage unit form for ease of administration and uniformity of dosage. The expression "dosage unit form" as used herein refers to a physically discrete unit of an inventive composition appropriate for the subject to be treated. It will be understood, however, that the total daily usage of the disclosed compositions will be decided by the attending physician within the scope of sound medical judgment. For any active agent, the therapeutically effective dose may be estimated initially either in cell culture assays or in animal models, usually mice, but also potentially from rats, rabbits, dogs, or pigs.

In a preferred embodiment, a disclosed composition is or is part of a pharmaceutical composition. As formulated with an appropriate pharmaceutically acceptable carrier in a desired dosage, such a pharmaceutical composition provided herein may be administered to humans and other mammals topically such as ocularly, nasally, bucally, orally, rectally, parenterally, intracisternally, intravaginally, or intraperitoneally. As used herein, the term "pharmaceutically acceptable carrier" includes any and all solvents, diluents, or other liquid vehicles, dispersion or suspension aids, surface active agents, isotonic agents, thickening or emulsifying agents, preservatives, solid binders, lubricants and the like, as suited to the particular dosage form desired. Remington's Pharmaceutical Sciences Ed. By Gennaro, Mack Publishing, Easton, PA, 1995, provides various carriers used in formulating pharmaceutical compositions and techniques for the preparation thereof. Some examples of materials which can serve as pharmaceutically acceptable carriers include, but are not limited to, sugars such as glucose and sucrose; excipients such as cocoa butter and suppository waxes; oils such as peanut oil, cottonseed oil, safflower oil, sesame oil, olive oil, corn oil, and soybean oil; glycols such a propylene glycol; esters such as ethyl oleate and ethyl laurate; agar; buffering agents such as magnesium hydroxide and aluminum hydroxide; alginic acid; pyrogen-free water; isotonic sahne; Ringer's solution; ethyl alcohol; and phosphate buffer solutions, as well as other non-toxic compatible lubricants such as sodium lauryl sulfate and magnesium stearate, as well as coloring agents, releasing agents, coating agents, preservatives and antioxidants can also be present in the composition, according to the judgment of the formulator. Liquid dosage forms for ocular, oral, or other systemic administration include, but are not limited to, pharmaceutically acceptable emulsions, microemulsions, solutions, suspensions, syrups and elixirs. In addition to the active agent(s), the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the ocular, oral, or other systemically-delivered compositions can also include adjuvants such as wetting agents, and emulsifying and suspending agents. Dosage forms for topical or transdermal administration of an inventive pharmaceutical composition include ointments, pastes, creams, lotions, gels, powders, solutions, sprays, inhalants, or patches. The active agent is admixed under sterile conditions with a pharmaceutically acceptable carrier and any needed preservatives or buffers as may be required. For example, ocular or cutaneous routes of administration are achieved with aqueous drops, a mist, an emulsion, or a cream. Administration may be therapeutic or it may be prophylactic. The disclosure includes ophthalmological devices, surgical devices, audiological devices or products which contain disclosed compositions (e.g., gauze bandages or strips), and methods of making or using such devices or products. These devices may be coated with, impregnated with, bonded to or otherwise treated with a composition as described herein. Transdermal patches have the added advantage of providing controlled delivery of the active ingredients to the body. Such dosage forms may be made by dissolving or dispensing the compound in the proper medium. Absorption enhancers can also be used to increase the flux of the compound across the skin. The rate can be controlled by either providing a rate controlling membrane or by dispersing the compound in a polymer matrix or gel. Compositions for rectal or vaginal administration are preferably suppositories which can be prepared by mixing the active agent(s) of this invention with suitable non-irritating excipients or carriers such as cocoa butter, polyethylene glycol or a suppository wax which are solid at ambient temperature but liquid at body temperature and therefore melt in the rectum or vaginal cavity and release the active agent(s). Solid dosage forms for oral administration include capsules, tablets, pills, powders, and granules. In such solid dosage forms, the active agent is mixed with at least one inert, pharmaceutically acceptable excipient or carrier such as sodium citrate or dicalcium phosphate and/or a) fillers or extenders such as starches, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. Solid compositions of a similar type may also be employed as fillers in soft and hard-filled gelatin capsules using such excipients as milk sugar as well as high molecular weight polyethylene glycols and the like. The solid dosage forms of tablets, dragees, capsules, pills, and granules can be prepared with coatings and shells such as enteric coatings, release controlling coatings and other coatings suitable for pharmaceutical formulation. In such solid dosage forms the active agent(s) may be admixed with at least one inert diluent such as sucrose or starch. Such dosage forms may also comprise, as is normal practice, additional substances other than inert diluents, e.g., tableting lubricants and other tableting aids such as magnesium stearate and microcrystalline cellulose. In the case of capsules, tablets and pills, the dosage forms may also comprise buffering agents. They may optionally contain pacifying agents and can also be of a composition that they release the active agent(s) only, or preferentially, in a certain part of the intestinal tract, optionally, in a delayed manner. Examples of embedding compositions which can be used include polymeric substances and waxes.

Further, an individualized cancer therapy is described which are present for illustration purpose only wherein the method is based on a genome editing system that targets specifically mutations or sequence differences present within a group of cancerous or pre-cancerous cells to induce its death or depletion, comprising the steps
a) Isolation of tumor sample(s) from a cancer subject;
b) Identification of cancer specific somatic mutations or sequence differences to determine a signature of the subject;
c) Design of a nucleic acid or molecule directed against one or more somatic mutation(s) or sequence difference(s) identified in b), and
d) Providing genome editing therapy based on c) to the cancer subject
e) Inducing of specific genome editing consequently causing cell death in cancerous or pre-cancerous cells.

Preferably, in step e) of the individualized cancer therapy, healthy cells are not largely affected, preferably not affected at all.

A somatic mutation as disclosed herein is a change in the DNA sequence of a somatic cell of a multicellular organism with dedicated reproductive cells; that is, any mutation that occurs in a cell other than a gamete, germ cell, or gametocyte.

In a preferred embodiment, a method comprises genome sequence analysis using the sample(s) or specimen obtained from the subject, preferable whole genome sequencing, and determination of unique cancer specific somatic mutations or sequence differences absent in healthy cells.

In a preferred embodiment, step d) of the individualized cancer therapy according to the present invention includes administering a mixture of CRISPR/Cas9 complexes to a subject. Each CRISPR/Cas9 complex within the treatment mixture contains a guide RNA molecule that will only recognize and bind with the somatic mutations or sequence differences according to step b) which are uniquely present in the genomic DNA of the cancerous or pre-cancerous cells.

In a preferred embodiment of the individualized cancer therapy method one or more homozygous or heterozygous somatic mutations or sequence differences according to b) are located within or close to a PAM, preferably on tumor driver genes and/or genes relevant for cell survival and/or proliferation, preferably but not limited to PAX5, MYC, mTOR or CD79B. It is further preferred that the nucleic acid sequence(s) according to b) are located at +2 to +7 position upstream of PAM and/or somatic mutations or sequence differences creating new PAMs.

In a preferred embodiment the individualized cancer therapy method comprises the additional steps
f) *In vitro* cultivation of cancerous or pre-cancerous cells from the sample(s) obtained from the cancer subject,
g) Adding the genome editing therapy obtained in step d) to the cultivated class of cells,
h) Determining of whether the genome editing therapy specifically induces cell death in the group of cancerous or pre-cancerous cells.

In a preferred embodiment of this method the group of cancerous or pre-cancerous cells is B lymphocytes.

In a preferred embodiment of this method the genome editing therapy according to d) is a genome editing tool, preferably a CRISPR-Cas complex comprising a guide RNA and a Cas endonuclease.

In a preferred embodiment of this method , the subject is a mammal, preferably a human.

Further, a method for treating cancer is described including administering to a subject a transport system obtained or obtainable according to a method as described herein wherein the Cas endonuclease or the sequence, i.e. the nucleic acid, encoding the Cas endonuclease and a guide RNA or the sequence, i.e. the nucleic acid, encoding the guide RNA, that targets the Cas endonuclease to the identified nucleic acid sequence that is specific for the "subgroup of the class of cells", i.e. the cancerous or pre-cancerous cells.

According to preferred embodiments of the method to treat cancer, the Cas endonuclease may be delivered as a protein complexed with the guide RNA, delivered as a DNA that encodes the Cas endonuclease to be transcribed in cells of the subject, or delivered as an mRNA to be translated in cells of the subject. In such embodiments, the guide RNA contains a targeting sequence that is complementary to the identified nucleic acid sequence that is specific for the subgroup of the class of cells, i.e. the cancerous or pre-cancerous cells.

Another aspect refers to a method for treating cancer in a subject, the method comprising:
a) sequencing a nucleic acid found in a healthy cell of a subject, thereby obtaining a wild-type sequence;
b) sequencing the respective nucleic acid found in a cancerous or pre-cancerous cell of the subject, thereby obtaining a mutated sequence;
c) comparing the wild-type sequence and the mutated sequence, thereby determining a difference between the wild-type sequence and the mutated sequence;
d) administering to the subject a single CRISPR/Cas complex, preferably a single CRISPR/Cas9 complex, or a mixture of CRISPR/Cas complexes, preferably a mixture of CRISPR/Cas9 complexes, whose guide RNA hybridize to fusion sequences of the genome that are in a cancerous or pre-cancerous cell but not in a healthy cell;
wherein the single CRISPR/Cas complex, preferably the single CRISPR/Cas9 complex, or the mixture of CRISPR/Cas complexes, preferably the mixture of CRISPR/Cas9 complexes, target cancer specific fusion sequences and generate double strand breaks inducing cell death; or wherein the single CRISPR/Cas complex, preferably the single CRISPR/Cas9 complex, or the mixture of CRISPR/Cas complexes, preferably the mixture of CRISPR/Cas9 complexes, target cancer specific fusion sequences and incorporate a protein coding gene sequence that results in the expression of a lethal protein and thereby induces cell death; or wherein the single CRISPR/Cas complex, preferably the single CRISPR/Cas9 complex, or the mixture of CRISPR/Cas complexes, preferably the mixture of CRISPR/Cas9 complexes, target cancer specific fusion sequences and incorporate a protein coding gene sequence that results in the expression of a protein that becomes expressed and represents a marker cell surface protein; wherein the mutated sequence is preferably located within or close to a PAM, more preferably the mutated sequence is located at +2 to +7 position upstream of PAM or mutation-derived PAM.

Another aspect is an *in-vitro* method, for inducing cell death in cancerous or pre-cancerous cells according to the claims, comprising the steps:
a) isolation of a class of cells from one or more sample(s) obtained from a subject, whereby healthy cells and cancerous or pre-cancerous cells are obtained;
b) identifying a nucleic acid sequence that is specific for the cancerous or pre-cancerous cells of the class of cells;
c) design of guide RNA(s) and/or DNA and/or protein directed against said nucleic acid sequence;
d) cloning of one or more sequence(s) encoding the guide RNA(s) and a Cas endonuclease into a viral system or a vector system, preferably into a viral vector, or including the guide RNA(s) and a Cas endonuclease into a transport system;
e) in vitro cultivation of the class of cells from the sample obtained from the subject;
f) adding the viral, the vector or the transport system obtained in step d) to the cultivated class of cells obtained in step e), and
g) optionally, determining if cell death is induced in the cancerous or pre-cancerous cells of the class of cells only,
wherein the nucleic acid sequence according to b) includes one or more homozygous and/or heterozygous mutation(s), preferably located on genes that are relevant for cell survival and/or proliferation; and wherein the one or more mutation(s) is or are located within or close to a PAM, preferably the one or more mutation(s) is or are located at +2 to +7 position upstream of PAM or mutation-derived PAM.

The method is an *in-vitro* method or combined with an *in-silico* method.

Preferred features of the aspects can be applied to all aspects of the present disclosure if applicable. For example, preferred features in view of the "subject" provided above can also be applied to the aspect related to the method for treating cancer as described above.

It is preferred that the nucleic acid sequence that is specific for a "subgroup of the class of cells" is located on a tumor driver gene, and/or is located on genes that are relevant for cell survival and/or proliferation. In Table 1 (Tab. 1) a list of proteins whose genes mutations could be targeted for cell death induction is presented.

**Tab. 1 List of proteins whose genes mutations could be targeted for cell death induction**

| |
|---|
| **Proteins regulating cell proliferation:** |
| RRAGC, POU2F2, CD79B, BTK, HIST1H1E, JMJD6, APP, TNF, DIABLO, AIFM1, CYCS, FAS, GSN, ROCK1, FASLG, TNFRSF1A, MCL1, FGF2, TGFB1, SPTAN1, FN1, MAP3K7, GAS2, TP53AIP1, DAPK1, RIPK1, CRADD, DAXX, TNFSF12, TNFSF10, TP53, EDARADD, CFLAR, TNFRSF14, TNFSF15, MDM2, EDAR, DFFA, APAF1, BAD, DFFB, PARP1, HUWE1, NGF, NGFR, BID, BIK, BAK1, ELANE, TNFRSF6B, FADD, BAX, CHEK2, CASP8AP2, AKT1, TRAF2, NOXA1, BIRC2, MAP3K5, BIRC3, XIAP, BIRC5, ELN, CASP2, TNFRSF10D, CASP3, MMP2, ACIN1, TNFRSF10C, CASP4, TNFRSF10B, CTSB, TNFRSF10A, CASP7, CASP8, HTRA2, CASP9, BCL2, CASP10, FAF1, SERPINA1, MAPK8, BCL2L1, BCL2L11, ATM, BCL2L2, TRADD, TNFRSF25, ENDOG, BBC3, BOK, CXCR1, MAP2K7, PIM2, GNA13, CREBBP, RHOA, CHD8, GNAI2, EZH2, CDC73, IRF8, PTPN6, ANKRD17, EBF1, SOCS1, B2M, PAX5, XPO1, PIK3R1, SGK1, TAF1, ACTB, CD22, SF3B1, FOXO1, SYK, ZBTB7A, YY1, MTOR, MYC, BCL6, BCL7A, TGFBR2, STAT6 |
| **Proteins regulating cell cycle:** |
| MOB1B, SPRED1, RB1, CHEK2, SFN, ATR, PPP2CA, CDKN2C, CHFR, CDKN2D, ESPL1, SMAD4, PTEN, TSC1, BRCA1, CDKN1B, CDKN1A, FOXO3, TFDP1, MAPKAPK5, TP53, GADD45B, MED13, TP53AIP1, SPRY2, TP53BP1, HUS1, NF1, TSC2, CDT1, LATS1, FOS, AKT1S1, CCND1, CDK6, FBXO5, MTOR, MYC, PPP3R1, AURKA, CCND3, BIRC5, CCNA1, TTK, JUN, CDC45, MAPRE1, , FZR1, NPAT, MAPK1, CCND2, CCNB2, RPTOR, KRAS, PTTG1, CCNC, CDC25B, RHEB, NEK2, CENPA, SKP2, NBN, PDPK1, BUB1, BUB3, RPS6KB1, TGFB1, CDK4, PLK1, GSK3B, CDCA3, XRCC6, MLST8, CDC7, BRAF, MAP2K1, CDK1, CCNA2, CDC6, CDK7, CCNB1, PRKDC, MAPK3, MAPK14, AKT1, NET1, CDC25C, MNAT1, MDM2, BUB1B, BTRC, INCENP, RAF1, MED12, NPM1, LMNB1, CDK8, CDC20, SP1, DBF4, CDC14A, CCNE2, CCNE1, CDC25A, CHEK1, AURKB, PPP3R1, CLSPN, TGFB3, HIPK2, HBP1, RB1, MED13, SMAD4, ZBTB17, TGFB1, GADD45A, ATM, GSK3B, ATR, CCNH, MAPK14, TBP, DHCR24, SMAD2, CHEK2, BBC3, SP1, SUV39H1, MAD1L1, MAD2L1, CCNB3, CENPE, TP53BP1, WEE1, TGFBR1, CDC7, CDK2, SMAD3, TGFBR2, TGFB2, PPP2CA, CDK1, PDPK1, CCND3, FBXO5, BRCA1, BRAF, LMNB1, XRCC6, CCNB1 |
| **Proteins regulating cell repair:** |
| MSH2, MSH3, CHEK1, NAMPT, SAFB, ABRAXAS1, UBE2N, AKT1S1, NBN, STK11, XRCC1, XRCC2, XRCC3, XRCC4, XRCC5, EIF4E, RPA1, MSH6, EIF4EBP1, CDC25A, TP53, TP53BP1, MTOR, EXO1, PARP1, INSR, DCLRE1C, MLH1, ARID2, IGF1, IGF1R, POLB, KAT2A, CHEK2, BRIP1, TERF2, IRS1, LIG3, LIG4, PRKDC, CDK2, TERF2IP, SESN1, ATR, ATRX, RELA, TADA2A, MDC1, H2AFX, PARP2, NHEJ1, BRCA1, FANCD2, RPS6, BRCA2, RPTOR, XRCC6, RPS6KB1, TSC1, TSC2, ACTB, RHEB, IRS2, NMNAT1, MRE11, PNKP, MDM2, UIMC1, ACTL6B, KAT2B, BRCC3, BARD1, TADA3, ACTL6A, FANCI, PTEN, AKT1, ARID1A, PDPK1, SESN2, PALB2, RAD50, INS, RAD51, DDIT4, SMARCA4, SMARCB1, SMARCC1, MYSM1, ATM, WRN, SMARCC2, SMARCD1, RAD52, SIRT1, SMARCD2, SMARCD3, FEN1 |
| **mTOR Signalling:** |
| BCL2L1, YY1, ATG13, TP53, SKP1, KCNJ11, GSK3B, MLST8, RRAGD, MAPT, RBX1, ATG14, SOS1, SLC2A4, SLC2A5, BTRC, TERT, MAF1, PRKCA, EIF4E, ULK1, EIF4EBP1, EIF4EBP2, INSR, AMBRA1, MTOR, MAPK1, IGF1, IGF1R, RHOA, IRS1, MAP2K1, RPTOR, DEPTOR, TFEB, IQGAP1, TSC1, LAMTOR3, FOXO3, TSC2, CUL1, RPS6, RPS6KA1, SREBF1, RPS6KA2, RPS6KB1, IRS2, RPS6KB2, DDIT4L, NFATC4, PXN, PPARG, RHEB, LAMTOR1, PPARGC1A, KRAS, LPIN1, RRAGA, DAP, ULK2, DDIT4, SGK1, CLIP1, PDPK1, GRB2, STAT3, AKT1S1, GRB10, LAMTOR2, RRAGC, SHC1, RAF1, RRAGB, PTEN, AKT1, TBC1D7, INS, STK11, TORC2, V-ATPase, PP2A, microtubule, F-actin. |
| **Necrosis:** |
| TNNT2, CASP3, MKI67, DIABLO, LTF, IL1RL1, HIF1A, CPB1, EIF2S1, MTOR, EIF2AK3, SMAD3, STAT1, TNNT2, ILF3, PTGS2, SEMA4D, GNLY, MIR375, F3, FGA, CASP3, APOE, EDN2, GJB1, MPC1, COPS8, LRP8, WNT10A, AQP4, PTP4A3, FAS, MMP2, m_Ctsq, GRK2, HIF1A, SMN1, NPAS4, PLIN1, KRAS, ALOX15B, F3, G6PC3, TCF12, CX3CR1, KIT, TK2, COL13A1, STAT3, BIRC5, COPS8, ALK, SLC8B1, MMP2, SMN1, ASIC2. |
| **PI3K_RAC1 Signalling:** |
| PIK3CA, RPS6KB1, PIK3CB, WASF2, PIK3CG, PIK3R1, PIK3R2, TIAM1, DOCK1, NCF2, BAIAP2, MAP2K4, SWAP70, PAK1, PTK2, PREX1, PIK3R3, GAB1, INPP5D, RAC1, PDPK1, RALBP1, CDC42, VAV1, RHOA, MAPK14, MAP3K1, PTEN, AKT1, RALGDS, ROCK1, PIK3C2A, IQGAP1, PIK3C2B, SRC, PIK3C3. |
| **TNF-alpha_TNFRSF1A Signalling:** |
| RIPK1, BAG4, TAB1, JUN, TRADD, MAPKAPK2, NFKBIA, MAP2K4, CREB1, MAPK8, TNF, MAPK9, MAPK10, MLKL, MAPK14, MAP3K1, RIPK3, PGAM5, MAP2K3, MAP3K5, CEBPB, FADD, MAP2K6, MAP2K7, TNFRSF1A, MAP3K14, MAPKAPK3, TRAF2, MAP3K7, TRAF5, CASP8, FOS. |
| **WNT Canonical Signalling:** |
| GBP1, CBY1, DKK1, CSNK1A1, AXIN1, SMARCA4, CSNK1E, BCL9, SENP2, CSNK2A1, GSK3B, APC, FRAT1, CREBBP, APCDD1, CTNNB1, CER1, SFRP1, WIF1, CYLD, TCF4, BTRC, LEF1, DVL1, NDP, LRP5, DVL2, NLK, NFATC1, CTNNB1, CAMK2A, CDC42, LEF1, MAP3K7, CREB1, WNT5A, ITPR1. |
| **NF-kB non-Canonical Signalling:** |
| TNFRSF13C, TNFSF13, CAMLG, REL, LTB, TNFRSF17, ICAM1, NFKB1, RELA, TNFRSF11A, NFKB2, RELB, LTBR, CHUK, TNF, TNFRSF13B, TNFSF13B, BIRC2, TNFRSF1A, CD40, MAP3K14, CD40LG, TRAF2, CXCL12, TNFSF11, MAP3K7, TRAF3, TRAF6. |
| **NOTCH Receptors Signalling:** |
| SNW1, HEY1, CDKN1A, JAG2, CIR1, CTBP1, RBPJ, FURIN, FBXW7, NEDD4, SPEN, NOTCH3, KDM5A, ADAM17, JAG1, AP2A1, DTX1, MAML1, HEY2, HES1, NCOR2, HDAC1, SLC35D2, CCND1, NOTCH1, MIB1, NOTCH2, NOTCH4, NUMB. |
| **Ras Signalling:** |
| TIAM1, CNKSR1, RASSF1, PARD3, PAK1, PGGT1B, RIN1, BRAF, RCE1, FNTA, PLCE1, TERT, ITPR1, RAC1, ABL1, PDPK1, PRKCZ, RALBP1, CDC42, SPHK1, MAPK1, RALGPS1, AFDN, ZDHHC9, RHOA, MAP3K1, RAF1, MAP2K1, MAP3K3, AKT1, GOLGA7, RALA, RALGDS. |
| **TGF-beta Signalling:** |
| ZFYVE9, RPS6KB1, CDKN1A, YY1, TGFBR2, CDKN2B, TGFBR1, SKIL, RHEB, TAB1, SMAD2, SMAD3, MAP2K4, TGFB3, ATF2, SMAD4, FOXH1, SERPINE1, CREBBP, SMAD6, SMAD7, PAK1, CDK1, SOS1, BRAF, CEBPB, GADD45B, FZR1, SP1, TRAF6, FOS, DAXX, RAC1, SMURF2, MTOR, PDPK1, GRB2, CDC42, JUN, MAPK1, LTBP1, MAPK3, SHC1, PARD6A, RHOA, MAPK14, MAP3K1, MAP2K1, MAP2K2, AKT1, MAP2K3, MAP3K5, MAP2K6, TGFB1, ELK1, TGFB2, XIAP, ROCK1, MAP3K7, LEFTY1, TSC2, RPS6. |
| **MERTK Signalling:** |
| TULP1, TIAM1, DOCK1, RASGRF1, MERTK, TUB, PAK1, PTK2, DIAPH1, SOS1, TNK2, CRK, GAS6, PRKCB, FOS, ITPR1, LGALS3, RAC1, CLIP1, IL6, MTOR, PDPK1, RALBP1, CDC42, GRB2, CXCL8, JUN, MAPK1, MAPK3, VAV1, PLCG2, SHC1, RHOA, TNF, MAP3K1, RAF1, BCAR1, MAP2K1, AKT1, RALGDS, ROCK2, IQGAP1, SRC. |
| **FOXO1 Signalling:** |
| IRS2, EGF, PPARG, EGFR, GADD45A, KRAS, SOD2, G6PC, PTK2, SOS1, GAB1, SH2B1, SGK1, INSR, IL6, IL6R, IL6ST, JAK1, JAK2, SIRT1, PDPK1, SH2B2, GRB2, STAT3, BCL2L11, CCND2, MAPK1, MAPK3, IGF1R, SHC1, IRS1, RAF1, MAP2K1, MAP2K2, AKT1, ERBB2, PCK2, MTDH, CASP3, INS, CCND1, XPO1, FOXO1, SRC. |
| **Receptors and Adaptor Proteins related to the cancer:** |
| EFNA3, EFNB2, EFNB3, EGF, HGF, NRG1, FGF19, ZAP70, EGFR, ALK, ESR1, NCK1, FGF1, KITLG, PTK2, FGF2, FGF6, FGF7, FGF8, CRK, PTN, EPHA2, FGFR1, GAS6, CRKL, BDNF, FGFR3, AR, FGFR2, FGFR4, PTPN11, SH2B1, FRS2, SYK, NTF3, NTF4, AREG, NTRK1, NTRK2, NTRK3, IGF1, IGF1R, DDR2, NGEF, VAV1, IGF2, IRS1, RET, HBEGF, FGF18, TGFA, COL3A1, KIT, FGF17, GAB2, DDR1, SRC, COL5A2, GDNF, PIK3CA, IRS2, PROS1, PDGFD, PDGFC, FLT3, PIK3R1, FLT3LG, COL11A1, AXL, FYN, MERTK, PDGFA, PDGFB, GAB1, EPHA1, PDGFRA, EPHA3, EPGN, EPHA4, TYK2, EPHA7, PDGFRB, MDK, EPHA8, EPHB1, BTC, COL2A1, EPHB2, EPHB3, EPHB4, IL6, IL6R, IL6ST, JAK1, JAK2, JAK3, STAT1, PLAU, GRB2, PLAUR, GRB7, STAT3, BTK, GRB10, PECAM1, STAT5A, RAPGEF1, EFNB1, STAT5B, PLCG1, PLCG2, SHC1, CD38, IL11, IL11RA, ERBB2, ERBB3, ERBB4, LYN, INS, EREG, MET, ROS1, EFNA1, EFNA2. |
| **Hippo_YAP1 Signalling:** |
| PIP3, PI3K, FZD, WNT, LRP6, DVL3, NF2, MIR372, TEAD2, RASSF2, TRIO, RASSF4, MIR373, TAOK1, ILK, RUNX2, AXIN1, SMAD1, TP73, SAV1, YAP1, TEAD4, ARHGEF25, WWTR1, DCAF1, APC, TEAD1, GNA11, CBL, RASSF1, RHOA, SPN, SMAD7, LATS1, RASSF3, PPP1R12A, AKT1, GNAQ, LATS2, STK3, STK4, SCRIB, DVL1, LRP5, MOB1A, DVL2. |
| **Cancer associated proteins resisting to cell death:** |
| BCL2L1, BCL2L2, PMAIP1, TP53, CFLAR, NFKB1, HIF1A, NFKBIA, FAS, MCL1, HIPK2, DAPK1, WWOX, BIRC6, MITF, TNFRSF10B, TNFRSF10A, FAF1, STAT3, BCL2L11, BID, TRADD, IGF1, BAK1, IGF1R, BIRC7, BAX, CTNNB1, AKT1, DCC, BIRC2, CASP1, BIRC3, XIAP, XAF1, BIRC5, CASP3, CASP4, CASP5, CASP6, CASP7, CASP8, CASP9, FOXO3, CASP10, BCL2, BCL2A1. |
| **Proteins regulating caspase activity:** |
| LMNB1, TP53, ENDOG, ARHGEF3, CFLAR, APP, NFKB1, HIF1A, GSN, HTRA2, GAS2, ACIN1, NTN1, BIRC6, MITF, PARP1, BIRC8, APAF1, DFFA, DFFB, STAT3, BID, DIABLO, BIRC7, CYCS, BAX, CTNNB1, DCC, BIRC2, CASP1, BIRC3, XIAP, XAF1, BIRC5, LMNA, CASP3, CASP4, SPTAN1, CASP5, CASP6, CASP7, ROCK1, CASP8, AIFM1, CASP9, CASP10. |
| **Proteins regulating Histone modification:** |
| BRCA1, CDC73, BMI1, PAF1, DZIP3, RNF2, CTR9, RING1, RNF20, LEO1, ATM, VRK1, RPS6KA4, RPS6KA5, CBX5, H2AX, AURKB, CHUK, RPS6KA3, KAT2A, CDK1, HDAC1, CDK2, STK4, AURKA, ATR, AURKC, SIRT1, MECP2, KPNA2, RBBP4, SIN3A, CREBBP, RBBP7, KAT5, KAT2B, EP300, NCOR1, SAP30, NCOR2, HDAC2, NCOA3, NCOA1, SAP18, DNMT1, ZBTB33, KDM4A, KDM5B, DNMT3A, SETDB1, DNMT3B, ZHX1, GFI1B, KDM2B, KDM1A, KDM3A, PCNA, KDM6B, SUV39H1, UHRF1, MBD1, EHMT2, SAE1, TP53, PML, HSF1, UBE2I, SUMO3, SUMO2, SUMO1, RCOR1, HDAC4, RANBP2, RANGAP1, PIAS1, SP100, UBA2. |

In Table 2 **(Tab. 2)** guide RNA sequences (CRISPR-derived RNA (crRNA) sequences with a tracrRNA sequence) which have been used in the examples are presented.

**Tab. 2 Guide RNA sequences (crRNA + tracrRNA; the tracrRNA sequence is always constituted by "GUUUUAGAGC UAUGCU", as depicted below)**

| |
|---|
| Cr006 |
| RNA |
| Synthetic sequence |
| GAAGGGUGUG ACCGCAACGU GUUUUAGAGC UAUGCU |
| Cr009 |
| RNA |
| Synthetic sequence |
| GUUGCCGUAG AGCUUGCCCA GUUUUAGAGC UAUGCU |
| Cr012 |
| RNA |
| Synthetic sequence |
| AACACCUCGG AGGUCUACCA GUUUUAGAGC UAUGCU |
| Cr019a |
| RNA |
| Synthetic sequence |
| CCAAGGAGCU CAGGAUGUAA GUUUUAGAGC UAUGCU |
| Cr021 |
| RNA |
| Synthetic sequence |
| AAUUCUUGGC AGGUAAAGGC GUUUUAGAGC UAUGCU |
| Cr022 |
| RNA |
| Synthetic sequence |
| GGCAGCGCUA UAAUAAUAGG GUUUUAGAGC UAUGCU |
| Cr029 |
| RNA |
| Synthetic sequence |
| CAACUGCCCA CAGGAUCAUC GUUUUAGAGC UAUGCU |
| Cr030 |
| RNA |
| Synthetic sequence |
| cACCGAGGAC ACCUGCGUCA GUUUUAGAGC UAUGCU |
| Cr033 |
| RNA |
| Synthetic sequence |
| GGCCUUCACA GAGACGAGGC GUUUUAGAGC UAUGCU |

In Table 3 **(Tab. 3)** PAM sequences used in the examples are listed.

**Tab. 3 PAM sequences corresponding to each crRNA.**

| |
|---|
| Cr006 |
| DNA |
| Homo sapiens |
| AGG |
| Cr009 |
| DNA |
| Homo sapiens |
| TGG |
| Cr012 |
| DNA |
| Homo sapiens |
| GGG |
| Cr019a |
| DNA |
| Homo sapiens |
| AGG |
| Cr021 |
| DNA |
| Homo sapiens |
| AGG |
| Cr022 |
| DNA |
| Homo sapiens |
| GGG |
| Cr029 |
| DNA |
| Homo sapiens |
| CGG |
| Cr030 |
| DNA |
| Homo sapiens |
| CGG |
| Cr033 |
| DNA |
| Homo sapiens |
| CGG |

In Table 4 **(Tab. 4)** primer sequences used in the examples are listed.

**Tab. 4 Primer sequences corresponding to each crRNA.**

| |
|---|
| Cr029 P1 |
| DNA |
| Synthetic sequence |
| GTTCAGCCCT TGGAGAATGA |
| Cr029 P2 |
| DNA |
| Synthetic sequence |
| TGCCAGAGGA CAGAGAGATT A |
| Cr006 P3 |
| DNA |
| Synthetic sequence |
| GTTCAGCCCT TGGAGAATGA |
| Cr006 P4 |
| DNA |
| Synthetic sequence |
| TGCCAGAGGA CAGAGAGATT A |
| Cr012 P5 |
| DNA |
| Synthetic sequence |
| GTTCAGCCCT TGGAGAATGA |
| Cr012 P6 |
| DNA |
| Synthetic sequence |
| TGCCAGAGGA CAGAGAGATT A |
| Cr009 P7 |
| DNA |
| Synthetic sequence |
| GTTCAGCCCT TGGAGAATGA |
| Cr009 P8 |
| DNA |
| Synthetic sequence |
| TGCCAGAGGA CAGAGAGATT A |
| Cr021 P9 |
| DNA |
| Synthetic sequence |
| GTTCAGCCCT TGGAGAATGA |
| Cr021 P10 |
| DNA |
| Synthetic sequence |
| TGCCAGAGGA CAGAGAGATT A |
| Cr022 P11 |
| DNA |
| Synthetic sequence |
| GTTCAGCCCT TGGAGAATGA |
| Cr022 P12 |
| DNA |
| Synthetic sequence |
| TGCCAGAGGA CAGAGAGATT A |
| Cr030 P13 |
| DNA |
| Synthetic sequence |
| GTTCAGCCCT TGGAGAATGA |
| Cr030 P14 |
| DNA |
| Synthetic sequence |
| TGCCAGAGGA CAGAGAGATT A |
| Cr019a P15 |
| DNA |
| Synthetic sequence |
| GTTCAGCCCT TGGAGAATGA |
| Cr019a P16 |
| DNA |
| Synthetic sequence |
| TGCCAGAGGA CAGAGAGATT A |
| Cr033 P17 |
| DNA |
| Synthetic sequence |
| GTTCAGCCCT TGGAGAATGA |
| Cr033 P18 |
| DNA |
| Synthetic sequence |
| TGCCAGAGGA CAGAGAGATT A |

The present invention is further illustrated by the following examples, sequences and figures from which further features, embodiments, aspects and advantages of the present invention may be taken. Further, the materials, methods, and examples are illustrative only and are not intended to be limiting unless otherwise specified.
**SEQ ID NO 1 (crRNA Cr006 + tracrRNA)**
   GAAGGGUGUG ACCGCAACGU GUUUUAGAGC UAUGCU
**SEQ ID NO 2 (crRNA Cr009 + tracrRNA)**
   GUUGCCGUAG AGCUUGCCCA GUUUUAGAGC UAUGCU
**SEQ ID NO 3 (crRNA Cr012 + tracrRNA)**
   AACACCUCGG AGGUCUACCA GUUUUAGAGC UAUGCU
**SEQ ID NO 4 (crRNA Cr019a + tracrRNA)**
   CCAAGGAGCU CAGGAUGUAA GUUUUAGAGC UAUGCU
**SEQ ID NO 5 (crRNA Cr021 + tracrRNA)**
   AAUUCUUGGC AGGUAAAGGC GUUUUAGAGC UAUGCU
**SEQ ID NO 6 (crRNA Cr022 + tracrRNA)**
   GGCAGCGCUA UAAUAAUAGG GUUUUAGAGC UAUGCU
**SEQ ID NO 7 (crRNA Cr029 + tracrRNA)**
   CAACUGCCCA CAGGAUCAUC GUUUUAGAGC UAUGCU
**SEQ ID NO 8 (crRNA Cr030 + tracrRNA)**
   CACCGAGGAC ACCUGCGUCA GUUUUAGAGC UAUGCU
**SEQ ID NO 9 (crRNA Cr033+ tracrRNA)**
   GGCCUUCACA GAGACGAGGC GUUUUAGAGC UAUGCU
**SEQ ID NO 10 (PAM to Cr006)**
   AGG
**SEQ ID NO 11 (PAM to Cr009)**
   TGG
**SEQ ID NO 12 (PAM to Cr012)**
   GGG
**SEQ ID NO 13 (PAM to Cr019a)**
   AGG
**SEQ ID NO 14 (PAM to Cr021)**
   AGG
**SEQ ID NO 15 (PAM to Cr022)**
   GGG
**SEQ ID NO 16 (PAM to Cr029)**
   CGG
**SEQ ID NO 17 (PAM to Cr030)**
   CGG
**SEQ ID NO 18 (PAM to Cr033)**
   CGG
**SEQ ID NO 19 (primer Cr029 P1)**
   GTTCAGCCCT TGGAGAATGA
**SEQ ID NO 20 (primer Cr029 P2)**
   TGCCAGAGGA CAGAGAGATT A
**SEQ ID NO 21 (primer Cr006 P3)**
   GTTCAGCCCT TGGAGAATGA
**SEQ ID NO 22 (primer Cr006 P4)**
   TGCCAGAGGA CAGAGAGATT A
**SEQ ID NO 24 (primer Cr012 P5)**
   GTTCAGCCCT TGGAGAATGA
**SEQ ID NO 25 (primer Cr012 P6)**
   TGCCAGAGGA CAGAGAGATT A
**SEQ ID NO 26 (primer Cr009 P7)**
   GTTCAGCCCT TGGAGAATGA
**SEQ ID NO 27 (primer Cr009 P8)**
   TGCCAGAGGA CAGAGAGATT A
**SEQ ID NO 28 (primer Cr021 P9)**
   GTTCAGCCCT TGGAGAATGA
**SEQ ID NO 28 (primer Cr021 P10)**
   TGCCAGAGGA CAGAGAGATT A
**SEQ ID NO 29 (primer Cr022 P11)**
   GTTCAGCCCT TGGAGAATGA
**SEQ ID NO 30 (primer Cr022 P12)**
   TGCCAGAGGA CAGAGAGATT A
**SEQ ID NO 31 (primer Cr030 P13)**
   GTTCAGCCCT TGGAGAATGA
**SEQ ID NO 32 (primer Cr030 P14)**
   TGCCAGAGGA CAGAGAGATT A
**SEQ ID NO 33 (primer Cr019a P15)**
   GTTCAGCCCT TGGAGAATGA
**SEQ ID NO 34 (primer Cr019a P16)**
   TGCCAGAGGA CAGAGAGATT A
**SEQ ID NO 35 (primer Cr033 P17)**
   GTTCAGCCCT TGGAGAATGA
**SEQ ID NO 36 (primer Cr033 P18)**
   TGCCAGAGGA CAGAGAGATT A
**SEQ ID NO 37 (crRNA Cr019a)**
   CCAAGGAGCU CAGGAUGUAA
**SEQ ID NO 38 (crRNA Cr021)**
   AAUUCUUGGC AGGUAAAGGC
**SEQ ID NO 39 (crRNA Cr022)**
   GGCAGCGCUA UAAUAAUAGG
**SEQ ID NO 40 (tracrRNA)**
   GUUUUAGAGC UAUGCU
**SEQ ID NO 41 (UniProtKB Q02548 (PAX5_HUMAN)**
**SEQ ID NO 42 (UniProtKB P01106 (MYC_HUMAN)** 
MDFFRVVENQQPPATMPLNVSFTNRNYDLDYDSVQPYFYCDEEENFYQQQQQSELQP
PAPSEDIWKKFELLPTPPLSPSRRSGLCSPSYVAVTPFSLRGDNDGGGGSFSTADQLEM
VTELLGGDMVNQSFICDPDDETFIKNIIIQDCMWSGFSAAAKLVSEKLASYQAARKDSGS
PNPARGHSVCSTSSLYLQDLSAAASECIDPSVVFPYPLNDSSSPKSCASQDSSAFSPSS
DSLLSSTESSPQGSPEPLVLHEETPPTTSSDSEEEQEDEEEIDVVSVEKRQAPGKRSES
GSPSAGGHSKPPHSPLVLKRCHVSTHQHNYAAPPSTRKDYPAAKRVKLDSVRVLRQISN
NRKCTSPRSSDTEENVKRRTHNVLERQRRNELKRSFFALRDQIPELENNEKAPKVVILKK
ATAYILSVQAEEQKLISEEDLLRKRREQLKHKLEQLRNSCA
**SEQ ID NO 43 (UniProtKB - P42345 (MTOR_HUMAN)** 
MLGTGPAAATTAATTSSNVSVLQQFASGLKSRNEETRAKAAKELQHYVTMELREMSQE
ESTRFYDQLNHHIFELVSSSDANERKGGILAIASLIGVEGGNATRIGRFANYLRNLLPSND
PVVMEMASKAIGRLAMAGDTFTAEYVEFEVKRALEWLGADRNEGRRHAAVLVLRELAIS
VPTFFFQQVQPFFDNIFVAVWDPKQAIREGAVAALRACLILTTQREPKEMQKPQWYRHT
FEEAEKGFDETLAKEKGMNRDDRIHGALLILNELVRISSMEGERLREEMEEITQQQLVHD
KYCKDLMGFGTKPRHITPFTSFQAVQPQQSNALVGLLGYSSHQGLMGFGTSPSPAKSTL
VESRCCRDLMEEKFDQVCQWVLKCRNSKNSLIQMTILNLLPRLAAFRPSAFTDTQYLQD
TMNHVLSCVKKEKERTAAFQALGLLSVAVRSEFKVYLPRVLDIIRAALPPKDFAHKRQKA
MQVDATVFTCISMLARAMGPGIQQDIKELLEPMLAVGLSPALTAVLYDLSRQIPQLKKDIQ
DGLLKMLSLVLMHKPLRHPGMPKGLAHQLASPGLTTLPEASDVGSITLALRTLGSFEFEG
HSLTQFVRHCADHFLNSEHKEIRMEAARTCSRLLTPSIHLISGHAHVVSQTAVQVVADVL
SKLLVVGITDPDPDIRYCVLASLDERFDAHLAQAENLQALFVALNDQVFEIRELAICTVGRL
SSMNPAFVMPFLRKMLIQILTELEHSGIGRIKEQSARMLGHLVSNAPRLIRPYMEPILKALIL
KLKDPDPDPNPGVINNVLATIGELAQVSGLEMRKWVDELFIIIMDMLQDSSLLAKRQVAL
 
WTLGQLVASTGYVVEPYRKYPTLLEVLLNFLKTEQNQGTRREAIRVLGLLGALDPYKHKV
NIGMIDQSRDASAVSLSESKSSQDSSDYSTSEMLVNMGNLPLDEFYPAVSMVALMRIFR
DQSLSHHHTMVVQAITFIFKSLGLKCVQFLPQVMPTFLNVIRVCDGAIREFLFQQLGMLVS
FVKSHIRPYMDEIVTLMREFWVMNTSIQSTIILLIEQIVVALGGEFKLYLPQLIPHMLRVFMH
DNSPGRIVSIKLLAAIQLFGANLDDYLHLLLPPIVKLFDAPEAPLPSRKAALETVDRLTESLD
FTDYASRIIHPIVRTLDQSPELRSTAMDTLSSLVFQLGKKYQIFIPMVNKVLVRHRINHQRY
DVLICRIVKGYTLADEEEDPLIYQHRMLRSGQGDALASGPVETGPMKKLHVSTINLQKAW
GAARRVSKDDWLEWLRRLSLELLKDSSSPSLRSCWALAQAYNPMARDLFNAAFVSCWS
ELNEDQQDELIRSIELALTSQDIAEVTQTLLNLAEFMEHSDKGPLPLRDDNGIVLLGERAA
KCRAYAKALHYKELEFQKGPTPAILESLISINNKLQQPEAAAGVLEYAMKHFGELEIQATW
YEKLHEWEDALVAYDKKMDTNKDDPELMLGRMRCLEALGEWGQLHQQCCEKWTLVND
ETQAKMARMAAAAAWGLGQWDSMEEYTCMIPRDTHDGAFYRAVLALHQDLFSLAQQCI
DKARDLLDAELTAMAGESYSRAYGAMVSCHMLSELEEVIQYKLVPERREIIRQIWWERLQ
GCQRIVEDWQKILMVRSLVVSPHEDMRTWLKYASLCGKSGRLALAHKTLVLLLGVDPSR
QLDHPLPTVHPQVTYAYMKNMWKSARKIDAFQHMQHFVQTMQQQAQHAIATEDQQHK
QELHKLMARCFLKLGEWQLNLQGINESTIPKVLQYYSAATEHDRSWYKAWHAWAVMNF
EAVLHYKHQNQARDEKKKLRHASGANITNATTAATTAATATTTASTEGSNSESEAESTEN
SPTPSPLQKKVTEDLSKTLLMYTVPAVQGFFRSISLSRGNNLQDTLRVLTLWFDYGHWP
DVNEALVEGVKAIQIDTWLQVIPQLIARIDTPRPLVGRLIHQLLTDIGRYHPQALIYPLTVAS
KSTTTARHNAANKILKNMCEHSNTLVQQAMMVSEELIRVAILWHEMWHEGLEEASRLYF
GERNVKGMFEVLEPLHAMMERGPQTLKETSFNQAYGRDLMEAQEWCRKYMKSGNVK
DLTQAWDLYYHVFRRISKQLPQLTSLELQYVSPKLLMCRDLELAVPGTYDPNQPIIRIQSIA
PSLQVITSKQRPRKLTLMGSNGHEFVFLLKGHEDLRQDERVMQLFGLVNTLLANDPTSL
RKNLSIQRYAVIPLSTNSGLIGWVPHCDTLHALIRDYREKKKILLNIEHRIMLRMAPDYDHL
TLMQKVEVFEHAVNNTAGDDLAKLLWLKSPSSEVWFDRRTNYTRSLAVMSMVGYILGLG
DRHPSNLMLDRLSGKILHIDFGDCFEVAMTREKFPEKIPFRLTRMLTNAMEVTGLDGNYR
ITCHTVMEVLREHKDSVMAVLEAFVYDPLLNWRLMDTNTKGNKRSRTRTDSYSAGQSV
EILDGVELGEPAHKKTGTTVPESIHSFIGDGLVKPEALNKKAIQIINRVRDKLTGRDFSHDD
TLDVPTQVELLIKQATSHENLCQCYIGWCPFW
**SEQ ID NO 44 (UniProtKB** - **P40259 (CD79B_HUMAN)**

MARLALSPVPSHWMVALLLLLSAEPVPAARSEDRYRNPKGSACSRIWQSPRFIARKRGF
TVKMHCYMNSASGNVSWLWKQEMDENPQQLKLEKGRMEESQNESLATLTIQGIRFEDN
GIYFCQQKCNNTSEVYQGCGTELRVMGFSTLAQLKQRNTLKDGIIMIQTLLIILFIIVPIFLLL
DKDDSKAGMEEDHTYEGLDIDQTATYEDIVTLRTGEVKWSVGEHPGQE

### BRIEF DESCRIPTION OF THE FIGURES

**Fig. 1** shows that CRISPR/Cas9 induced editing of PAX5, MYC and CD79B genes caused significant reduced OCI-Ly3 cell viability in comparison to cells treated with non-targeting control crRNA (NTC).
**Fig. 2** shows that the targeting of genomic alterations located in any arbitrary gene or at any gene region does not induce cancer cell death.
**Fig. 3** shows that knocking out of PAX5 gene using an OCI-LY3 specific-crRNA causes cell death.
**Fig. 4** shows that dual sgRNA-guided knock out of PAX5 targeting OCI-Ly3 specific mutations causes cell death.
**Fig. 5** shows that transcription factor MYC interacts with PAX5 and influences DLBCL development.
**Fig. 6** shows that knocking out PAX5 and MYC gene using a double-crRNAs strategy causes cell death.
**Fig. 7** shows CRISPR-Cas9 mediated PAX5-MYC rearrangement in B-cell lymphoma cells.
**Fig. 8** shows the absence of cancer cell death by targeting an intron using a single crRNA.
**Fig. 9** shows a method overview.

### EXAMPLES

### Methods

### Cell culture conditions

Diffuse large B-cell lymphoma (DLBCL) OCI-LY3 (ACC 761) and BJAB (ACC 757) cells were purchased from DSMZ. These cells have been previously used often as *in vitro* models for the discovery of novel cancer therapeutic agents. Cells were cultured according to manufacturer's protocol in RPMI 1640 Medium (ATCC modification) (A1049101; Thermo Fisher Scientific) supplemented with heat-inactivated 20% fetal bovine serum (FBS) (A3160502; Thermo Fisher Scientific), and 1% penicillin-streptomycin solution (10378016; Thermo Fisher Scientific) (referred to as complete medium). Cells were cultured and maintained in an incubator at 37°C and 5% CO₂.

### Guide RNA design

gRNA targeting mutations were designed using SNP-CRISPT tool (https://www.flyrnai.org/tools/snp _crispr/). Using this tool, multiple SNPs can be uploaded for the design of gRNAs targeting single or multiple nearby mutations simultaneously. It designs all possible gRNAs for one variant based on the positioning of PAM. Moreover, Custom Alt-R^{®} CRISPR-Cas9 guide RNA tool (IDT) was used. On-target and off-target scores were evaluated using this tool.

### Nucleofections

The ribonucleoprotein (RNP) complex was made according to the manufacturer's instructions. Briefly, each Alt-R crRNA (IDT) and Alt-tracrRNA-ATTO550 (1072533, IDT) was reconstituted to 100 µM with Nuclease-Free Duplex Buffer (11-05-01-14, IDT). crRNA and tracrRNA oligos were mixed at equimolar concentrations in a sterile PCR tube to a final duplex concentration of 44 µM. Oligos were annealed by heating at 95°C for 5 min in PCR thermocycler and the mix was slowly cooled to room temperature. The crRNA-tracrRNA duplex (sgRNA) and Alt-R^{®} S.p. HiFi Cas9 Nuclease 3NLS (1078727, IDT) were precomplexed by combining and gently mixing by resuspending and incubated at room temperature for 10-20 min. sgRNAs were complexed with Cas9 at a molar ratio between 1:1 to 3:3 (sgRNA:Cas9) to form ribonucleoproteins (RNPs), which was always freshly prepared. To improve transfection efficiency, Alt-R electroporation enhancer (IDT) was added to the mix at a final concentration of 1.75 µM. About 5 x 10⁵ cells were resuspended in Neon electroporation buffer R and electroporated using the 10 µl Neon transfection system kit, with two pulses at 1400 V, width 20 ms. Transfected cells were incubated 48-72 h in pre-warmed RPMI-1640 medium supplemented with 20% FBS (A3160502; Thermo Fisher) (complete medium without antibiotics).

### Determination of editing efficiency and gene deletion/recombination

Cells were lysed and genomic DNA was extracted using QuickExtract^{™} DNA Extraction Solution (Lucigen; Biozym QE09050). For each target, PCR primers were designed to amplify the region flanking the targeted gDNA, generating a PCR amplicon of <900 bp. To screen for the presence of indels/mutations in the genomic region of interest, PCR was performed using GoTaq^{®} G2 Hot Start Taq Polymerase (M5122; Promega). PCR amplicon was purified using PureLink^{®} PCR Purification Kit (Invitrogen; K3100-02) or NucleoSpin Gel and PCR Clean-up kit (740609.250; Macherey-Nagel).

Sanger sequencing was then performed through a commercial vendor (Eurofins; Heidelberg) with one of the two primers used for amplification. The sequence traces obtained from Sanger sequencing were analysed by Interference of CIRPSR Edits (ICE), a tool that calculates overall editing efficiency and determines the profiles of all different types of CRISPR edits. By utilizing the indel patterns and their relative ratios provided by ICE default parameters, the control (NTC) trace sequence and a gene specific sgRNA edited trace sequence were aligned, allowing for the visualization of the indel patterns of each polyclonal population.

PCR was used to analyse the experiments where two different RNPs targeting two unique regions of the same or different gene thereby leading to a deletion and cancer specific chromosomal rearrangement. A confirmation of the presence of deletions could be obtained also by designing primers spanning the region to be deleted. In dual gRNA edited cells, the PCR band is only amplified upon successful editing by both crRNA. Without dual editing and subsequent rearrangement, the primers would be incapable of generating any PCR product.

### Cell viability assay

Immediately post nucleofection, cells were plated in a 96-well plate in a total of 100 µL medium containing RealTime-Glo MT cell viability reagents (G9711;RealTime-Glo MT Cell Viability Assay; Promega). Cells were incubated in a 37°C, 5% CO₂-humidified incubator (Thermo Fisher Scientific). After one hour incubation, the plate was read on a SpectraMax^{®} iD3 multi-mode microplate reader (Molecular Devices) set at 37°C to determine luminescence. Reading was taken again from the same plate at 72 h post nucleofection. The luminescence at 72h was normalized to 1h to compensate for any variations derived from pipetting.

### Statistical analysis

Statistical analysis was performed using Graphpad Prism software (GraphPad Software Inc., San Diego, CA, USA). For multiple group comparisons, ANOVA followed by Turkeys, Dunnett's or Sidak's multiple comparison test was performed depending on the data to be compared. Statistical tests and number of repetitions are described in the legends. Bar plots show mean ± standard error mean (SEM).

### Example 1: crRNAs as positive controls for cell death

### Purpose

These experiments were started to show that knockout of functional driver genes reduces cancer cell viability. Genes that can be targeted include but are not limited to PAX5, MYC and CD79B using CRISPR/Cas9 using non-mutational crRNAs. Given that these genes are often mutated in lymphoma cells, and are implicated in multiple signalling pathways critical for lymphoma cell functions and survival (Reddy et al. 2017), it was examined whether their depletion are functionally impairing the survival of lymphoma cells particularly diffuse large B-cell lymphoma (DLBCL) cells. Furthermore, these genes are often mutated in OCI-Ly3 cell lines. From whole exome sequencing analysis of OCI-Ly3 cells, 65 mutations in PAX5, 4 mutations in MYC, and 4 mutations in CD79B were identified in OCI-Ly3 cell lines. These mutations were validated via Sanger sequencing. The reduced cell viability upon CRISPR/Cas9 mediated gene depletion can serve as the positive control for further experiments.

### Experiment

PAX5, MYC and CD79B genes were targeted using gRNA guiding the CRISPR/Cas9 complex specifically to early exons (non-mutational regions). Nucleofections were performed to deliver the ribonuclear protein complex (RNP) into the OCI-Ly3. Genomic DNA was isolated from cells and screened for the presence of site-specific gene modification by PCR followed by Sanger sequencing. Editing efficiency was determined using Interference of CRISPR edits (ICE) analysis (Synthego).

### Results

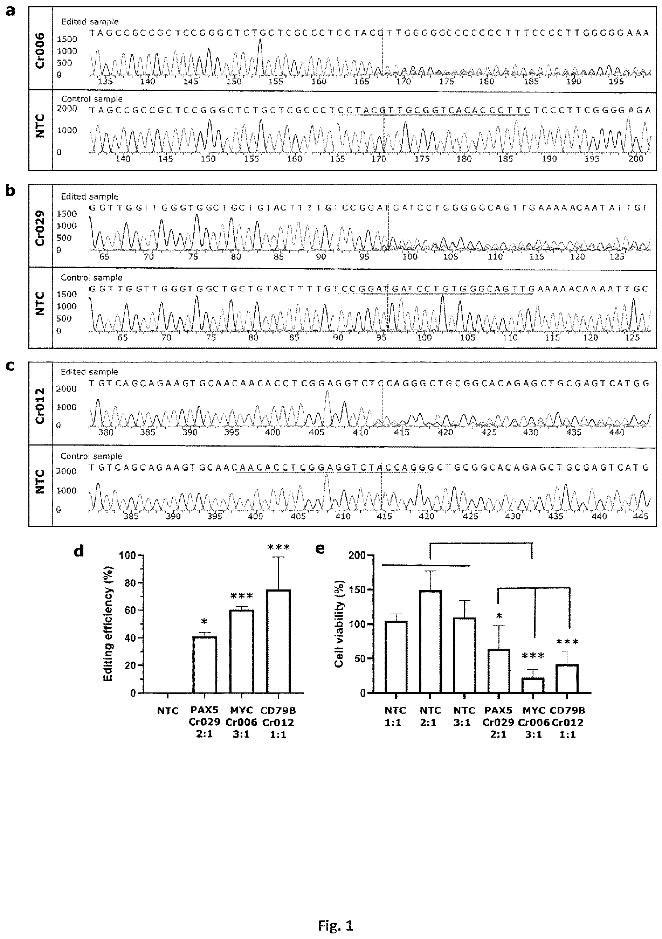

CRISPR/Cas9 induced editing of these genes caused significant reduced OCI-Ly3 cell viability in comparison to cells treated with non-targeting control crRNA (NTC) **(****Fig. 1****).**

**Fig. 1****:** knockout of functional driver genes induces cell death in OCI-Ly3 cells. OCI-LY3 lymphoma cells (2,5 x105 cells) were electroporated with Cr006 (targeting early exon of MYC gene), Cr029 (targeting early exon of PAX5 gene) and Cr012 (targeting early exon of CD79B gene). After 72 h treatment, cells were collected, genomic DNA was isolated. The target sequence regions were amplified with primers flanking the expected edited point followed by Sanger sequencing of the amplicon. The traces show the edited and control (non-edited) sanger traces in the area around the guide RNA binding site(s). The horizontal black underlined region represents the guide sequence, along with the dotted gray underlined PAM site. The cut site is depicted by a vertical black dotted line (a-c). Total editing efficiencies were then calculated by comparison of the sequencing chromatogram of test sample with control cells treated with non-targeting crRNA (NTC) using the ICE web tool. Results are shown as mean ± SEM; * p < 0.05, *** p < 0.001 (d). Cell viability was determined as percentage in viability from the negative control in OCI-LY3 cells electroporated with Cr006, Cr029 and Cr012. Results are shown as mean ± SEM; * p < 0.05, *** p < 0.001 (e).

### Discussion:

The results suggest that survival and depletion of the driver genes may be exploited as a novel strategy in lymphoma treatment by interfering with tumour growth and survival through pathways not affected by standard therapies. Moreover, these results can serve as positive controls for editing and cell death in the further experiments.

### Example 2: crRNAs as negative controls for cell death

### Purpose

This example provides evidence to show that not every gene deficiency causes cell death. Two major points are:
1. Depletion of one gene may cause cell death in one cancer cell and not in another.
2. The positioning of the guideRNA is a decisive factor for cell death induction.

CD79B was used as an example for point 1 and POU2F2 was used as an example for point 2.

### Experiment

CD79B or POU2F2 genes were targeted using gRNA guiding the CRISPR/Cas9 complex specifically to early and late exons (non-mutational regions) respectively. Nucleofections were performed to deliver the ribonuclear protein complex (RNP) into the OCI-Ly3 and/or BJAB cells. Genomic DNA was isolated from cells and screened for the presence of site-specific gene modification by PCR followed by Sanger sequencing. Editing efficiency was determined using Interference of CRISPR edits (ICE) analysis (Synthego).

### Results

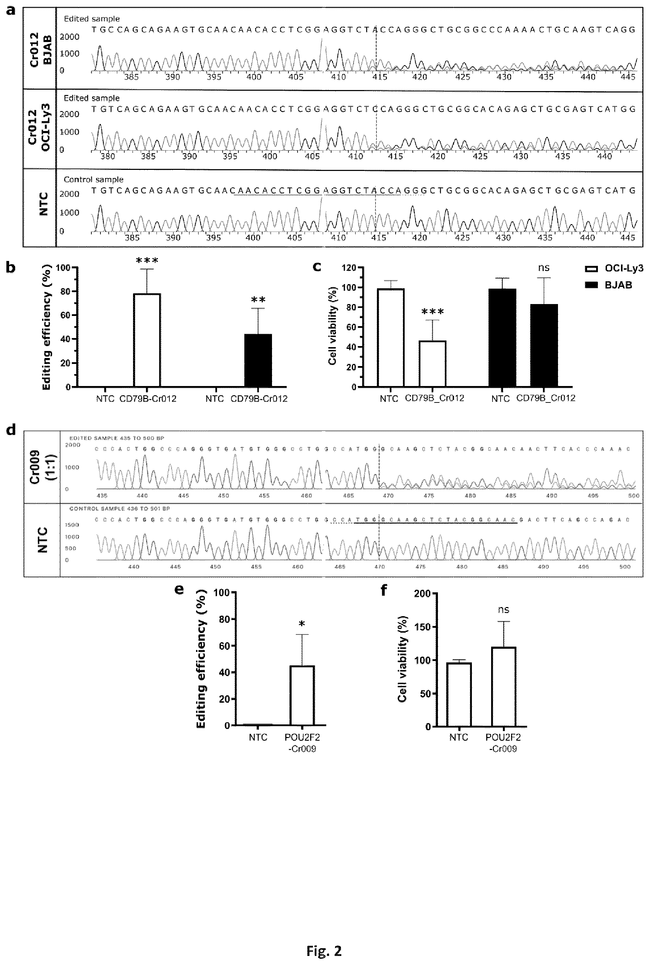

Cr012 targeting early exon of CD79B causes gene editing and thereby gene deficiency in both OCI-Ly3 and BJAB cells, although the efficiency is higher in the former cell line. This editing caused significant cell death in OCI-Ly3 but not in BJAB cells indicating that CD79B is critical for the survival of OCI-Ly3 and not BJAB (Fig. 2 (a-c)).

Cr009 was directed towards a non-mutated region within a late exon (9/14) of the POU2F2 gene. The DNA-binding transcription factor POU2F2 regulates cytokine and immunoglobulin expression. As expected, significant gene editing was induced in target B cells, while no cell death induction was measured, demonstrating that targeting late exons of a driver gene does not always result in cancer cell death **(****Fig. 2** **(d-e)).**

**Fig. 2****:** Targeting of genomic alterations located in any arbitrary gene or at any gene region does not induce cancer cell death. Cr012 was designed to target a non-mutated early exon of CD79B. OCI-LY3 and BJAB lymphoma cells (2,5 x10⁵ cells) were electroporated with crRNA. After 72 h treatment, cells were collected, genomic DNA was isolated, and target sequence regions were amplified and sequenced. Gene editing efficiency was ascertained by comparison of Sanger sequences with control cells treated with non-targeting crRNA (NTC). In the traces, the horizontal black underlined region represents the guide sequence, along with the dotted gray underlined PAM site. The cut site is depicted by a vertical black dotted line **(a, b).** Cell viability of target cells compared to control cells was determined in a cell viability assay **(c).** Cr009 directed towards a non-mutated late exon of POU2F2 was used as negative control for cell death in OCI-LY3 lymphoma cells (crRNA: Cas9 ratio 1:1) **(d-f).** Results are shown as mean ± SEM and are representative of two independent experiments.

### Discussion:

The results indicate that different cell lines show different gene specific dependencies. Therefore, this shows the importance of target gene library preparation and screening for each cancer cell line. It is critical to target early exons or exons coding functionally critical protein domains. These results also show that not all editing or depletion of gene cause cell death indicating that the cell death is not a non-specific event due to stress caused by DSBs.

### Example 3: Targeting single mutation in a driver gene

### Purpose

In this approach the goal is to induce cell death specifically in OCI-Ly3 cells by using a single mutation specific crRNA. 65 mutations in PAX5 were identified from whole exome sequencing analysis of OCI-Ly3 cells. 46 out of the 65 mutations have PAM proximity and can be targeted by gRNA. crRNA named Cr021 was used for proof of concept experiments. Cr021 is complementary to one of the mutations having PAM proximity and being a homozygous mutation in early exon of PAX5 gene that is found only in OCI-Ly3 cancer cells and not in wild-type PAX5 genes in normal (i.e. non-cancerous) genome. A BJAB cell line was used as control which is heterozygous for the mutation.

The mutation was targeted utilizing a CRISPR/Cas gene editing tool to disable the PAX5 gene in OCI-Ly3 cells. The CRISPR/Cas9-grRNA complex aligns with the variant containing target gene, which enables it to execute a double-stranded DNA break. This action is followed by an attempt by the cell to reclose scission, most often through a process known as nonhomologous end joining (NHEJ). The reclosure is often imperfect and faulty as a single or multiple nucleotides are mostly lost or sometimes inserted during the process resulting in a genetic frameshift and the subsequent production of non-functional transcripts, a gene knockout of PAX5.

### Experiment

OCI-Ly3 and BJAB cells were nucleofected with ribonucleoprotein (RNP) complex consisting of Cas9 protein and gRNA (Cr021 gRNA). 72h post nucleofections cell viability assays were performed using RealTime-Glo MT Cell Viability Assay. Genomic DNA was isolated from cells and screened for the presence of site-specific gene modification by PCR followed by Sanger sequencing. Editing efficiency was determined using Interference of CRISPR edits (ICE) analysis (Synthego).

### Results

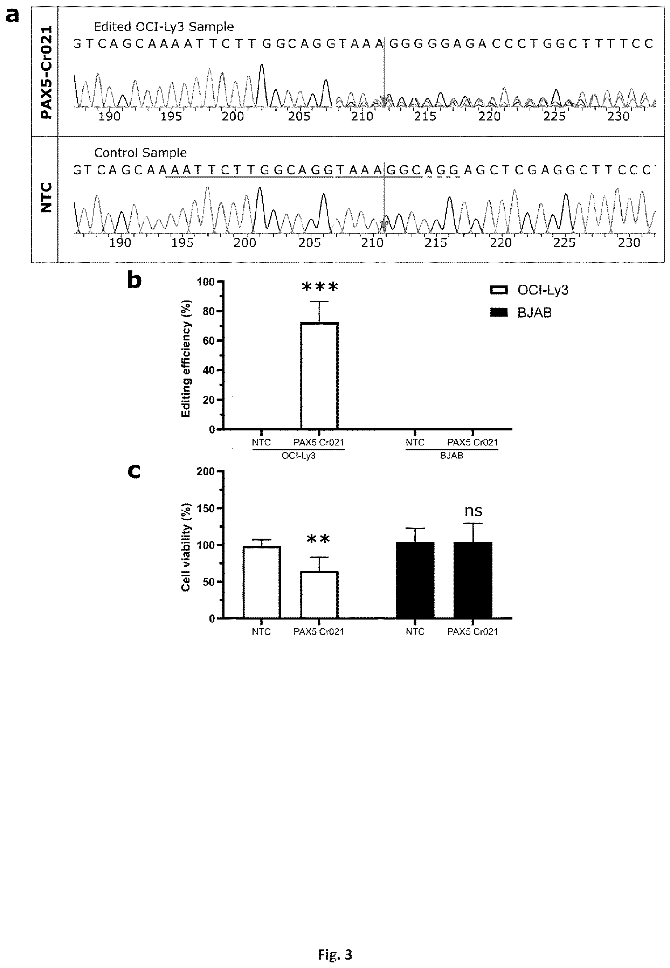

Cr021 showed efficient editing of ~80 % with a nearly similar knock-out score as estimated via ICE analysis of sequencing Sanger electropherograms of the PCR products flanking the gRNAs. The editing and consequent knockout of PAX5 resulted in reduced cell viability compared to that of non-targeting control crRNA (NTC) in OCI-Ly3 cells. However, no editing and cell death was observed in BJAB cells which is heterozygous for the mutation **(****Fig.3****).**

**Fig. 3****:** knocking out of PAX5 gene using an **OCI-LY3 specific**-crRNA causes cell death. OCI-LY3 and BJAB lymphoma cells (2,5 x105 cells) were electroporated with Cr0021 (targeting exon 2 intron junction). After 72 h treatment, cells were collected, genomic DNA was isolated. The target sequence regions were amplified with primers flanking the expected edited point followed by Sanger sequencing of the amplicon. The traces show the edited and control (non-edited) sanger traces in the area around the guide RNA binding site(s). The horizontal black underlined region represents the guide sequence, along with the dotted gray underlined PAM site. The cut site is depicted by a vertical black dotted line **(a).** Total editing efficiencies were then calculated by comparison of the sequencing chromatogram of test sample with control cells treated with non-targeting crRNA (NTC) using the ICE web tool. Results are shown as mean ±SEM **(b).** Cell viability was determined as percentage in viability from the negative control in OCI-LY3 and BJAB lymphoma cells electroporated with Cr021, ±SEM **(c).**

### Discussion:

The results indicate that CRISPR/Cas9 complex can be used to target a specific mutation in early exons in cancer cells and induce cancer specific cell death. Cells devoid or heterozygous to the mutation are not susceptible to editing and cell death. Cell death can be induced using a single gRNA provided the mutation is present in the early exons of a gene. These finding highly alleviates the potential of CRISPR/Cas9 as a individualized therapy for cancer without having the adverse side effects on healthy cells. It has also been demonstrated that targeting driver genes including PAX5 in B-cell lymphomas, specifically DLBCL, has immense therapeutic potential.

### Example 4: Targeting two different mutations in a single driver gene

### Purpose

In this embodiment, a strategy was used to maximize therapeutic potential. One of the limitations of Example 3, is that not all the genes have mutations in early exons. More often these mutations are in the introns or late exons. To resolve this, the strategy was expanded to target more genes, with the ultimate goal of building a genome-wide library of sgRNAs targeting mutations for systematic genetic loss-of-function. Two different crRNAs targeting mutations in different parts of the gene were used. This enables to remove a big part of the gene or the complete gene thereby depleting the protein in the cells. To demonstrate this, two different crRNAs (Cr021 and Cr022) targeting different regions of PAX5 were used. Cr021 (is in exon 2 intron junction) targets a homozygous mutation in OCI-Ly3 cells and is heterozygous in BJAB cells, while Cr022 (is in the last exon) targets a homozygous mutation in OCI-Ly3 cells and is absent in BJAB cells.

### Experiment

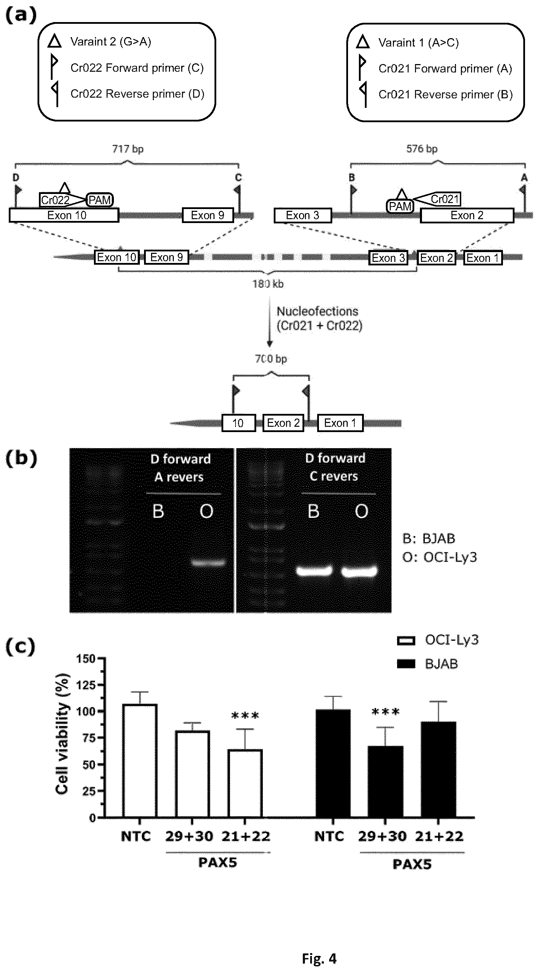

OCI-Ly3 and BJAB cells were nucleofected with a combination of crRNAs, i.e. Cr021 and Cr022. 72h post nucleofections cell viability assays were performed using RealTime-Glo MT Cell Viability Assay. Genomic DNA was isolated and screened for the presence of site-specific gene modification by PCR followed by Sanger sequencing. To validate the editing, PCR was performed for Cr021 and Cr022 combination with forward primer from Cr022 and reverse primer from Cr021 **(****Fig. 4a****).** In dual gRNA edited cells the PCR band is amplified only upon successful editing by both crRNA.

### Results

The genomic region flanking both target sites are amplified by PCR. The dual Cr021 and Cr022 edited cells favours the generation of strong loss-of-function alleles by creating large deletion of ~180 kbp. The editing is followed by recombination that bring both the edited genomic regions in proximity. Therefore, the presence of PCR band indicates editing by both crRNAs and absence indicates failure of editing at least by one crRNA **(****Fig. 4b****).** Editing was observed, as shown by PCR amplification, and reduced cell viability in OCI-Ly3 using dual gRNAs. However, no editing was observed in BJAB cells which consequently did not affect cell viability **(****Fig. 4c****).**

**Fig. 4****:** knocking out of PAX5 gene using **OCI-LY3 specific**-crRNAs causes cell death. To knock out PAX5 gene, two different crRNAs (Cr021 and Cr022) targeting of PAX5 were used. Schematic representation of the PAX5 gene-targeting strategy to generate a cell line-specific PAX5 knockout, including a map of the PAX5 exons, primers, and the position of Cr0021 (is in exon 2 intron junction) and Cr022 (is in the last exon) **(a).** OCI-LY3 and BJAB lymphoma cells (2,5 x105 cells) were electroporated with Cr021/Cr022 and Cr029/Cr030 (as a positive control for cell death and editing). After 72 h treatment, cells were collected, genomic DNA was isolated. The target sequence regions were amplified with primers flanking the expected edited point followed by agarose gel electrophoresis to show the edited samples. Left: Agarose gel electrophoresis shows PCR amplified products using the forward and reverse external primers (primer A and D) for Cr021 and Cr022. The PCR band is amplified just in double-crRNAs edited OCI-Ly3 cells (left gel). The PCR band is not amplified in BJAB, as cells are not edited. Left: Agarose gel electrophoresis shows PCR amplified products using the forward and reverse primers (primer C and D) flanking Cr022. Two PCR bands in OCI-Ly3 (O) and BJAB (B) samples, indicate the presence of genomic DNA in both samples **(b).** Cell viability was determined as percentage in viability from the negative control in OCI-LY3 (white *columns)* and BJAB (black *columns)* with electroporated with Cr021/Cr022 and Cr029/Cr030. Results are shown as mean ±SEM; *** p < 0.001 **(c).** Primers: A is P10; B is P9; C is P12 and D is P11.

### Discussion:

The results indicate that very specific and large gene deletions (~ 180kb) were induced in OCI-Ly3 cells using CRISPR/Cas9 by simultaneously introducing DSBs at two distinct cancer specific mutation sites. This approach expands the opportunity to induce large-scale deletions by targeting any variant-specific mutations (early exon, late exon, intron, or even intergenic) in the genes of interest thereby depleting the protein. This enhances the prospect to manipulate different target sites and thereby induce cancer-specific cell death. Using this strategy, vital functional domains of a protein can also be removed thereby rendering the protein non-functional.

### Example 5: Using variant specific crRNA directed towards two different genes

### Purpose

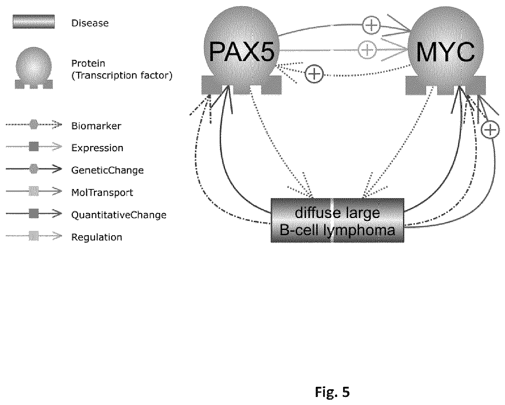

In this embodiment, a strategy was used to for inducing chromosomal rearrangements between different genes thereby making both genes non-functional. It is well known that there is a constant cross talk between several cellular pathways and associated proteins in cancer cells. Several proteins interact directly or indirectly with each other aiding in the development, survival and sustained growth of cancer cells. Therefore, CRISPR/Cas9 was used as a therapeutic approach to target multiple mutated gene that are involved in the growth and survival of cancer cells. In this example two genes were to be targeted, first, PAX5 as shown above and, second, MYC. MYC is a transcription factor, an oncogene that is dysregulated, translocated and mutated in several cancers including lymphomas. It is associated with very aggressive clinical behaviour. It is an interactor of PAX5 **(****Fig. 5****),** and has a similar role in B-cell lymphoma survival. Therefore, two distinct gRNAs, one targeting PAX5 variant and the other targeting MYC variant, were combined. This results in the editing and chromosome recombination resulting in simultaneous truncation of PAX5 and MYC rendering them functionally inefficient. This approach can be used to make large chromosomal deletions and rearrangements making multiple mutated gene dysfunctional thereby extending the target library for cancer therapy.

### Experiment

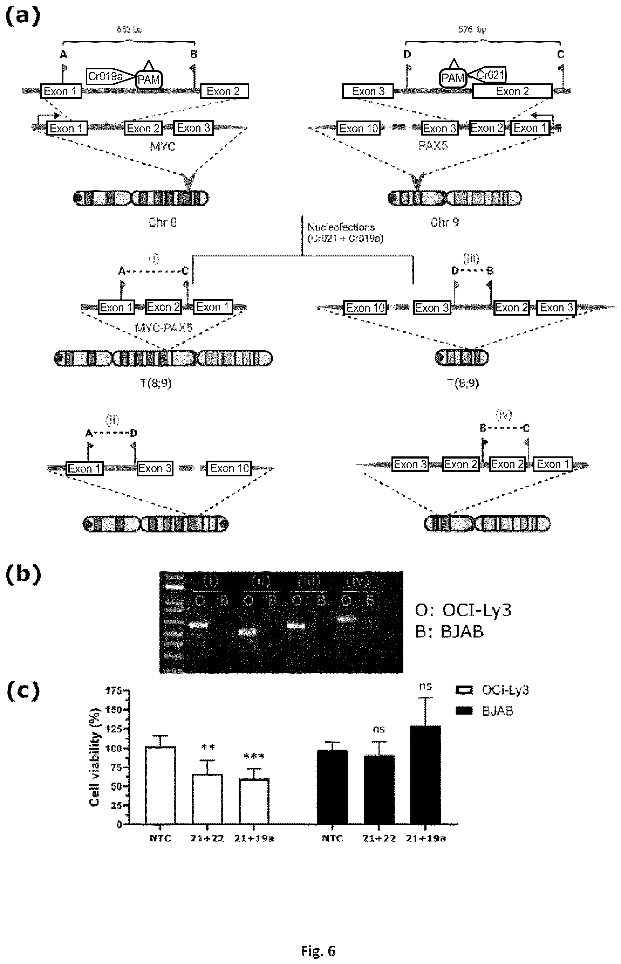

OCI-Ly3 and BJAB cells were nucleofected with a combination of crRNAs i.e. Cr021 and Cr019a targeting PAX5 (on Chromosome 9) and MYC (on chromosome 8), respectively. 72h post nucleofections cell viability assays were performed using RealTime-Glo MT Cell Viability Assay (Promega). Genomic DNA was isolated and screened for the presence of site-specific gene modification by PCR followed by Sanger sequencing. To validate the editing, PCR was performed for Cr021 and Cr019a combination with different sets of primers spanning Cr021 and Cr019a target site **(****Fig. 6(a)****).** In dual gRNA edited cells the PCR band is only amplified upon successful editing by both crRNA.

### Results

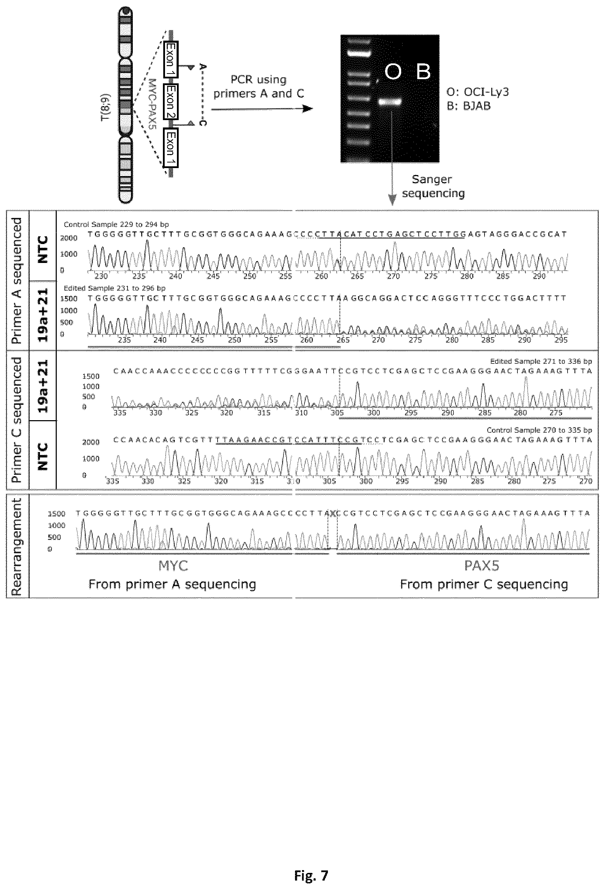

In human genome MYC variant (C>T), at position 127736999 is located on chromosome 8 and PAX5 variant (A>C), at position 37020625 is located on chromosome 9. The two target loci, i.e. PAX5 mutation and MYC mutation sites, are separated by a genomic sequence of 90.7 Mb. The editing is followed by recombination that bring both the edited genomic regions in proximity. The genomic region flanking both target sites were amplified by PCR. PCR analysis of the dual Cr021 and Cr019a edited cells demonstrated the induction of MYC-PAX5 rearrangement and of a large deletion of ~90.7 Mb between the two cut sites. Therefore, the presence of PCR band indicates editing by both crRNAs and absence indicates failure of editing at least by one crRNA **(****Fig. 6(b)****;** **Fig. 7****).** Editing was observed, as shown by PCR amplification and reduced cell viability in OCI-Ly3 using dual gRNAs targeting PAX5 and MYC. However, no editing was observed in BJAB cells which consequently did not affect cell viability **(****Fig. 6(c)****).**

**Fig. 6****:** knocking out PAX5 and MYC gene using a double-crRNAs strategy causes cell death. To knock out PAX5 and MYC gene, two different crRNAs (Cr021 and Cr019a) targeting respective genes were used. Schematic representation of the chromosomal rearrangements strategy to generate a cell line-specific PAX5 and MYC knockout, including a map of the PAX5 and MYC exons, primers, and the position of Cr0021 (targeting exon 2 intron junction) and Cr019a (intron1) **(a).** OCI-LY3 and BJAB lymphoma cells (2,5 x105 cells) were electroporated with Cr021 and Cr019a. After 72 h treatment, cells were collected, genomic DNA was isolated. The target sequence regions were amplified with primers flanking the expected edited point followed by agarose gel electrophoresis to show the edited samples. Agarose gel electrophoresis shows PCR amplified products using the forward and reverse external primers for 4 different chromosomal rearrangements possibility showing in a. The PCR band is amplified just in double-crRNAs edited OCI-Ly3(O) cells. The PCR band is not amplified in BJAB (B), as cells are not edited **(b).** Cell viability was determined as percentage in viability from the negative control in OCI-LY3 (white *columns)* and BJAB (black *columns)* cells electroporated with Cr021/Cr019a and Cr021/Cr022. Results are shown as mean ±SEM; ** p < 0.01, *** p < 0.001 **(c).** Primers: A is P15; B is P16; C is P10 and D is P9.

**Fig. 7****:** CRISPR-Cas mediated PAX5-MYC rearrangement in B-cell lymphoma cells. Schematic showing induction of PAX5-MYC rearrangement in OCI-Ly3 cells using CRISPR-Cas9 system. It includes position of primers 'A' and 'C' (Primers: A is P15, and C is P10) used to amplify the genomic loci using PCR (a). Agarose gel analysis image of PCR performed shows presence of PCR product in dual gRNA edited OCI-Ly3 (O) cells and not in control BLAB (B) cells (b). PCR amplicons were sequenced using Sanger sequencing. The traces show the edited sequences and control cells treated with non-targeting crRNA (NTC). The PAX5-MYC rearrangement traces confirms deletion of ~90 Mb sequence (grey X).

### Discussion:

The results indicate that a method for inducing cancer specific chromosomal rearrangement by targeting cancer mutations with a CRISPR/Cas9 complex is disclosed. This method may be used to perturb the function of multiple driver gene located on different chromosomes by targeting cancer specific mutations without affecting cells lacking these mutations. Using this method distinct cancer survival pathway networks can be manipulated. Overall, the susceptibility of the cancer cells can be significantly enhanced using this strategy.

### Example 6: Falsification experiments

### Purpose

The following non-inventive embodiments provide evidence that not every random mutation may be used as target to induce cancer cell death.

### Experiment

Concretely, the insufficiency of using (a) single crRNA targeting introns, (b) single crRNA targeting late exons, and (c) single crRNA targeting mutations located beyond the core-sequence was demonstrated.

### a) Single crRNA targeting introns

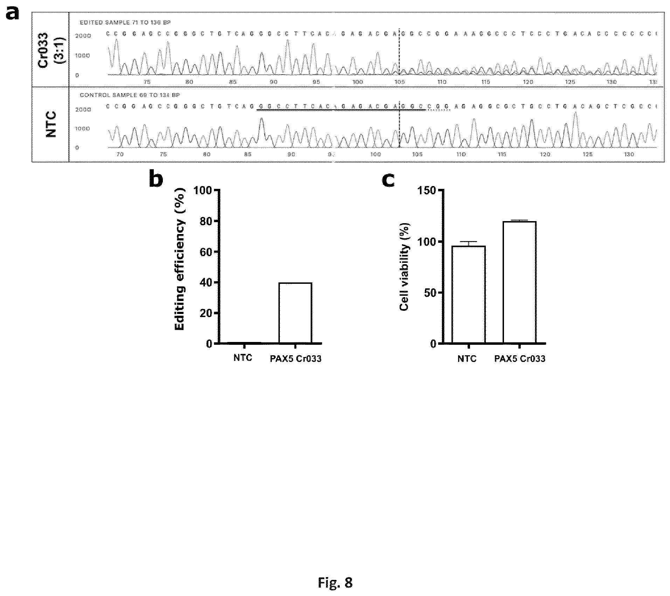

crRNA033 was directed towards an OCI-LY3-specific mutation located within the late intron of the PAX5 gene. 2,5 x10⁵ lymphoma cells were electroporated with 5 µM crRNA (crRNA:Cas9 ratio 3:1). After 72 h treatment, cell viability of target cells compared to control cells treated with non-targeting crRNA (NTC) was ascertained (RealTime-GloTM MT Cell Viability Assay, Promega). Afterwards, cells were harvested, genomic DNA isolation was performed, and target sequence regions were amplified and sequenced. Gene editing efficiency in target cells was determined by comparison of Sanger sequences with wild-type (control) cells (**Fig. 8 (a)**) (ICE Analysis, Synthego [Conant D et al. CRISPR J. 2022 Feb;5(1):123-130.]).

### b) Single crRNA targeting late exons

crRNA009 was directed towards a non-mutated region within a late exon (9/14) of the POU2F2 gene. The DNA-binding transcription factor POU2F2 regulates cytokine and immunoglobulin expression. 2,5 x10⁵ lymphoma cells were treated with 1,7 µM crRNA (crRNA:Cas9 ratio 1:1). After 72 h incubation, cell viability of target OCI-Ly3 cells compared to control cells treated with non-targeting crRNA (NTC) was ascertained (RealTime-GloTM MT Cell Viability Assay, Promega). Subsequently, lymphoma cells were harvested, genomic DNA isolation was carried out, and target sequence regions were amplified and sequenced. Gene editing efficiency in target cells was determined by comparison of Sanger sequences with wild-type (control) cells (ICE Analysis, Synthego Conant D et al. CRISPR J. 2022 Feb;5(1):123-130.]).

### Results

### a) Single crRNA targeting introns

Despite substantial gene editing (40% indel), no abatement of cell viability was observed upon treatment of target lymphoma cells with crRNA033 (Fig. 8). Thus, these experiments demonstrate the infeasibility of inducing DSBs within an intron of a tumor driver gene by means of a single crRNA.

### b) Single crRNA targeting late exons

Significant gene editing was induced in target B cells, while no cell death induction was measured, demonstrating that targeting late exons of driver does not always result in cancer cell death (Fig. 2 (d-f)).

Fig. 8: Targeting of genomic alterations located in introns did not lead to loss of function and eventually cancer cell death. Cr033 was designed to target an OCI-LY3-specific mutation found within an intron of the PAX5 gene. 2,5 x10⁵ lymphoma cells were electroporated with 5 µM crRNA (crRNA:Cas9 ratio 3:1). After 72 hours treatment, cells were harvested, genomic DNA isolation was performed, and target sequence regions were amplified and sequenced. Gene editing efficiency in target cells was determined by comparison of Sanger sequences with wild-type (control) cells. The horizontal black underlined region represents the guide sequence. The horizontal dotted underline is the PAM site. The cut site is depicted by a vertical black dotted line (a, b). Cell viability of target cells compared to control cells treated with non-targeting crRNA (NTC) was ascertained. Results are shown as mean ± SEM (c).

### Discussion:

The results indicate that the provided data of non-inventive embodiments represents evidence that not every random mutation may be used as target to induce cancer cell death. This embodiment also provides evidence that targeting altered genomic sequences of cancer cells to induce double strand breaks (DSBs) and thereby heightened DNA damage is not always sufficient to induce cancer cell death.

### METHOD OVERVIEW

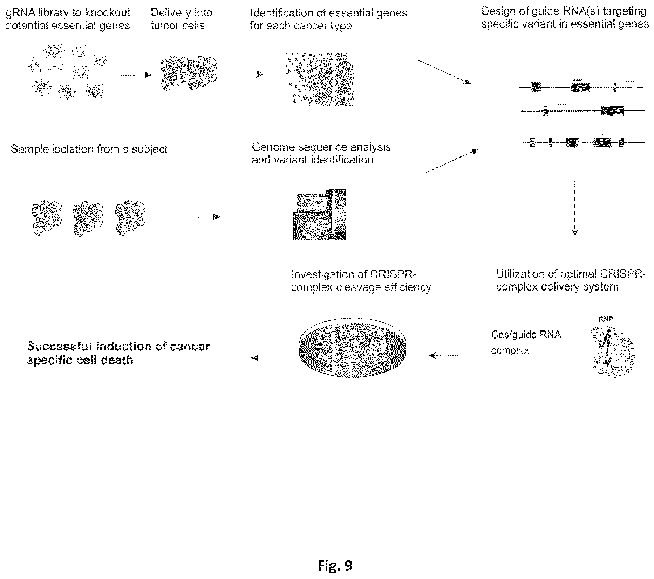

In **Fig. 9** a method overview according to the present invention is presented. First, a gRNA library is prepared for CRISPR-mediated knockout and identification of essential genes for each cancer type. After genome sequence analysis, variant-specific crRNAs are designed, synthesized, and cloned or included into an appropriate delivery system. Tumor cells are treated *in vitro* or *in vivo* with the variant-specific CRISPR-complex. crRNA cleavage efficiency and cancer cell death induction is determined. Finally, lead candidates are selected for the development of cancer-specific treatment strategies.

### IMPACT OF THE INVENTION

The present methods and compositions according to the present invention are applicable to several malignancies in a subject-specific manner, with the added crucial advantage of reducing side effects on healthy cells. The methods and compositions described in the different embodiments may be particularly advantageous in subjects with tumor relapse and refractory to standard therapies.

Further, the methods and compositions described herein allow for targeting of mutations regardless of their silent or non-silent nature. While current approaches are limited to non-silent and/or non-synonymous mutations resulting in amino acid changes, the present methods and inventions also take advantage of silent or synonymous mutations as therapeutic targets for cancer cell death induction.

Moreover, the present methods and compositions described herein comprise the design of crRNAs targeting genomic mutations located close to PAM, preferably at +2 to +7 position upstream of PAM, and/or mutations located within PAM. Hence, toleration of single nucleotide mismatches is avoided and enhanced specificity is provided.

In contrast to other methods such as DNA-based strategies, the use of the RNA-based CRISPR-Cas system includes the advantage that there is no risk of sequence integration into the subject's genome, avoiding substantial long-term, irreversible side effects. This is mainly due to the lability and short half-life of crRNAs and other forms of RNA within the bloodstream or in general within the human body.

### REFERENCES

Hanahan, D., & Weinberg, R. A. (2011). Hallmarks of Cancer: The Next Generation. Cell, 144(5), 646-674. https://doi.org/10.1016/j.cell.2011.02.013
Makita, S., Maruyama, D., & Tobinai, K. (2020). Safety and Efficacy of Brentuximab Vedotin in the Treatment of Classic Hodgkin Lymphoma. OncoTargets and Therapy, Volume 13, 5993-6009. https://doi.org/10.2147/OTT.S193951
Shankland, K. R., Armitage, J. O., & Hancock, B. W. (2012). Non-Hodgkin lymphoma. The Lancet, 380(9844), 848-857. https://doi.org/10.1016/S0140-6736(12)60605-9
Sung, H., Ferlay, J., Siegel, R. L., Laversanne, M., Soerjomataram, I., Jemal, A., & Bray, F. (2021). Global Cancer Statistics 2020: GLOBOCAN Estimates of Incidence and Mortality Worldwide for 36 Cancers in 185 Countries. CA: A Cancer Journal for Clinicians, 71(3), 209-249. https://doi.org/10.3322/caac.21660
Weinberg RA. (2014). The biology of cancer. Garland Science, Taylor & Francis Group, LLC.
Halwani, A. S., Panizo, C., Isufi, I., Herrera, A. F., Okada, C. Y., Cull, E. H., Kis, B., Chaves, J. M., Bartlett, N. L., Ai, W., de la Cruz-Merino, L., Bryan, L. J., Houot, R., Linton, K., Briones, J., Chau, I., von Keudell, G. R., Lu, H., Yakovich, A., ... Flowers, C. R. (2021). Phase 1/2 study of intratumoral G100 (TLR4 agonist) with or without pembrolizumab in follicular lymphoma. Leukemia & Lymphoma, 1-13. https://doi.org/10.1080/10428194.2021.2010057
Fricker, L. D. (2020). Proteasome Inhibitor Drugs. Annual Review of Pharmacology and Toxicology, 60(1), 457-476. https://doi.org/10.1146/annurev-pharmtox-010919-023603
Burslem, G. M., Schultz, A. R., Bondeson, D. P., Eide, C. A., Savage Stevens, S. L., Druker, B. J., & Crews, C. M. (2019). Targeting BCR-ABL1 in Chronic Myeloid Leukemia by PROTAC-Mediated Targeted Protein Degradation. Cancer Research, 79(18), 4744-4753. https://doi.org/10.1158/0008-5472.CAN-19-1236
Sheikh, I. N., Ragoonanan, D., Franklin, A., Srinivasan, C., Zhao, B., Petropoulos, D., Mahadeo, K. M., Tewari, P., & Khazal, S. J. (2021). Cardiac Relapse of Acute Lymphoblastic Leukemia Following Hematopoietic Stem Cell Transplantation: A Case Report and Review of Literature. Cancers, 13(22), 5814. https://doi.org/10.3390/cancers13225814
Sun, Y., Zhao, X., Ding, N., Gao, H., Wu, Y., Yang, Y., Zhao, M., Hwang, J., Song, Y., Liu, W., & Rao, Y. (2018). PROTAC-induced BTK degradation as a novel therapy for mutated BTK C481S induced ibrutinib-resistant B-cell malignancies. Cell Research, 28(7), 779-781. https://doi.org/10.1038/s41422-018-0055-1
Sheikh, I. N., Ragoonanan, D., Franklin, A., Srinivasan, C., Zhao, B., Petropoulos, D., Mahadeo, K. M., Tewari, P., & Khazal, S. J. (2021). Cardiac Relapse of Acute Lymphoblastic Leukemia Following Hematopoietic Stem Cell Transplantation: A Case Report and Review of Literature. Cancers, 13(22), 5814. https://doi.org/10.3390/cancers13225814
Marron, T. U., Kalac, M., & Brody, J. (2017). An Update on the Use of Immunotherapy in the Treatment of Lymphoma. Current Hematologic Malignancy Reports, 12(4), 282-289. https://doi.org/10.1007/s11899-017-0396-8
Wu, H., & Cao, C. (2019). The application of CRISPR-Cas9 genome editing tool in cancer immunotherapy. Briefings in Functional Genomics, 18(2), 129-132. https://doi.org/10.1093/bfgp/ely011
Xing, H., & Meng, L. (2020). CRISPR-cas9: a powerful tool towards precision medicine in cancer treatment. Acta Pharmacologica Sinica, 41(5), 583-587. https://doi.org/10.1038/s41401-019-0322-9
Ahmad, G., & Amiji, M. (2018). Use of CRISPR/Cas9 gene-editing tools for developing models in drug discovery. Drug Discovery Today, 23(3), 519-533. https://doi.org/10.1016/j.drudis.2018.01.014
Gallipoli, P., Giotopoulos, G., Tzelepis, K., Costa, A. S. H., Vohra, S., Medina-Perez, P., Basheer, F., Marando, L., di Lisio, L., Dias, J. M. L., Yun, H., Sasca, D., Horton, S. J., Vassiliou, G., Frezza, C., & Huntly, B. J. P. (2018). Glutaminolysis is a metabolic dependency in FLT3ITD acute myeloid leukemia unmasked by FLT3 tyrosine kinase inhibition. Blood, 131(15), 1639-1653. https://doi.org/10.1182/blood-2017-12-820035
Chen, Y., Wen, R., Yang, Z., & Chen, Z. (2021). Genome editing using CRISPR/Cas9 to treat hereditary hematological disorders. Gene Therapy. https://doi.org/10.1038/s41434-021-00247-9
Sahin U, Kreiter S, Diken M, Diekmann J, Koslowski M, Britten C, Castle J, Löwer M, Renard B, Omokoko T, & de Graaf J.H. (2012). Personalized vaccines for cancer (Patent No. WO/2012/159754).
Shuber A.P. (2018). Treating cancer with Cas endonuclease complexes (Patent No. WO/2018/175502).

## Claims

1. Method for the manufacture of individualized CRISPR/Cas complexes comprising the steps
a) identifying in a tumor specimen of a human cancer patient at least one of the following cancer specific mutations: mutation at position 127736999 on chromosome 8, mutation at position 37020625 on chromosome 9 and mutation at position 36840626 on chromosome 9
b) preparing for the at least one mutation identified in a), preferably for two or all mutations identified in a), an individualized CRISPR/Cas complex, wherein each individualized CRISPR/Cas complex comprises a guide RNA and a Cas endonuclease,
wherein the guide RNA for the individualized CRISPR/Cas complex for the mutation at position 127736999 on chromosome 8 comprises a crRNA according to SEQ ID NO 37 and a tracrRNA,
wherein the guide RNA for the individualized CRISPR/Cas complex for the mutation at position 37020625 on chromosome 9 comprises a crRNA according to SEQ ID NO 38 and a tracrRNA, and
wherein the guide RNA for the individualized CRISPR/Cas complex for the mutation at position 36840626 on chromosome 9 comprises a crRNA according to SEQ ID NO 39 and a tracrRNA,
wherein the tumor specimen is from B-cell-lymphocytes.

2. Method according to claim 1, wherein two or all of the mutations according to a) are identified and wherein two or all individualized CRISPR/Cas complexes according to b) are prepared.

3. Method according to claim 1 or 2, wherein each guide RNA is directed against the nucleic acid sequence that is specific for the mutation in cancerous or pre-cancerous cells.

4. Method according to any of the preceding claims, wherein the Cas endonuclease is a type I, type II or type III endonuclease, preferably a type I endonuclease, most preferably a CRISPR/Cas9 endonuclease.

5. *In-vitro* method for inducing cell death in cancerous or pre-cancerous cells of B-cell-lymphocytes, comprising the steps:
a) isolation of B-cell-lymphocytes from one or more specimen obtained from a human cancer patient or a human subject supposed to have or to develop cancer, whereby non-cancerous B-cell-lymphocytes and cancerous or pre-cancerous B-cell-lymphocytes are obtained;
b) cultivation, preferably *in-vitro* cultivation, of the B-cell-lymphocytes from the one or more specimen obtained in a);
c) identifying in the cancerous or pre-cancerous B-cell-lymphocytes at least one of the following cancer specific mutations: mutation at position 127736999 on chromosome 8, mutation at position 37020625 on chromosome 9 and mutation at position 36840626 on chromosome 9
d) preparing an individualized CRISPR/Cas complex for each of the cancer specific mutations identified in c) according to step b) of a method for the manufacture of individualized CRISPR/Cas complexes according to at least one of claims 1 to 4,
e) adding the one ore more prepared individualized CRISPR/Cas complexes to the cultivated B-cell-lymphocytes obtained in step b), and
f) optionally, determining if cell death is induced in the cancerous or pre-cancerous B-cell-lymphocytes of said cultivated B-cell-lymphocytes and preferably determining if cell death is not induced in the non-cancerous B-cell-lymphocytes of said cultivated B-cell-lymphocytes.

6. Composition comprising one or more individualized CRISPR/Cas complexes manufactured according to the method of any of claims 1 to 4.

7. Individualized CRISPR/Cas complex comprising a guide RNA and a Cas endonuclease,
wherein the guide RNA comprises a crRNA according to SEQ ID NO 37 and a tracrRNA,
wherein the guide RNA comprises a crRNA according to SEQ ID NO 38 and a tracrRNA, or
wherein the guide RNA comprises a crRNA according to SEQ ID NO 39 and a tracrRNA.

8. An individualized CRISPR/Cas complex manufactured according to the method of any of claims 1 to 4, a composition according to claim 6 or a CRISPR/Cas complex according to claim 7, for use in the treatment of B-cell-lymphoma.

9. An individualized CRISPR/Cas complex manufactured according to the method of any of claims 1 to 4, a composition according to claim 6 or a CRISPR/Cas complex according to claim 7, for use in inducing cell death in cancerous or pre-cancerous B-cell-lymphocytes.

## Patentansprüche

1. Verfahren zur Herstellung von individualisierten CRISPR/Cas-Komplexen, umfassend die Schritte:
a) Identifizieren mindestens einer der folgenden krebsspezifischen Mutationen in einer Tumorprobe eines menschlichen Krebspatienten: Mutation an Position 127736999 auf Chromosom 8, Mutation an Position 37020625 auf Chromosom 9 und Mutation an Position 36840626 auf Chromosom 9
b) Herstellung eines individualisierten CRISPR/Cas-Komplexes für die mindestens eine in a) identifizierte Mutation, vorzugsweise für zwei oder alle in a) identifizierten Mutationen, wobei jeder individualisierte CRISPR/Cas-Komplex eine Leit-RNA und eine Cas-Endonuklease umfasst,
wobei die Leit-RNA für den individualisierten CRISPR/Cas-Komplex für die Mutation an Position 127736999 auf Chromosom 8 eine crRNA gemäß SEQ **ID** NO 37 und eine tracrRNA umfasst,
wobei die Leit-RNA für den individualisierten CRISPR/Cas-Komplex für die Mutation an Position 37020625 auf Chromosom 9 eine crRNA gemäß SEQ **ID** NO 38 und eine tracrRNA umfasst, und
wobei die Leit-RNA für den individualisierten CRISPR/Cas-Komplex für die Mutation an Position 36840626 auf Chromosom 9 eine crRNA gemäß SEQ **ID** NO 39 und eine tracrRNA umfasst,
wobei die Tumorprobe aus B-Zell-Lymphozyten stammt.

2. Verfahren nach Anspruch 1, wobei zwei oder alle Mutationen gemäß a) identifiziert werden und wobei zwei oder alle individualisierten CRISPR/Cas-Komplexe gemäß b) hergestellt werden.

3. Verfahren nach Anspruch 1 oder 2, wobei jede Leit-RNA gegen die Nukleinsäuresequenz gerichtet ist, die für die Mutation in Krebs- oder präkanzerösen Zellen spezifisch ist.

4. Verfahren nach einem der vorstehenden Ansprüche, wobei die Cas-Endonuklease eine Typ-I-, Typ-II- oder Typ-III-Endonuklease ist, vorzugsweise eine Typ-I-Endonuklease, am bevorzugtesten eine CRISPR/Cas9-Endonuklease.

5. In-vitro-Verfahren zur Induktion des Zelltods in Krebs- oder präkanzerösen Zellen von B-Zell-Lymphozyten, umfassend die folgenden Schritte:
a) Isolierung von B-Zell-Lymphozyten aus einer oder mehreren Proben, die von einem menschlichen Krebspatienten oder einer Person entnommen wurden, bei der der Verdacht auf Krebs besteht oder die voraussichtlich an Krebs erkranken wird, wobei nicht-kanzeröse B-Zell-Lymphozyten und kanzeröse oder präkanzeröse B-Zell-Lymphozyten gewonnen werden;
b) Kultivierung, vorzugsweise In-vitro-Kultivierung, der B-Zell-Lymphozyten aus der oder den in a) gewonnenen Proben;
c) Identifizierung mindestens einer der folgenden krebsspezifischen Mutationen in den krebsartigen oder präkanzerösen B-Zell-Lymphozyten: Mutation an Position 127736999 auf Chromosom 8, Mutation an Position 37020625 auf Chromosom 9 und Mutation an Position 36840626 auf Chromosom 9;
d) Herstellung eines individualisierten CRISPR/Cas-Komplexes für jede der in c) identifizierten krebsspezifischen Mutationen gemäß Schritt b) eines Verfahrens zur Herstellung individualisierter CRISPR/Cas-Komplexe gemäß gemäß mindestens einem der Ansprüche 1 bis 4;
e) Hinzufügen des einen oder der mehreren hergestellten individualisierten CRISPR/Cas-Komplexe zu den in Schritt b) erhaltenen kultivierten B-Zell-Lymphozyten, und
f) optional, Bestimmen, ob in den krebsartigen oder präkanzerösen B-Zell-Lymphozyten der genannten kultivierten B-Zell-Lymphozyten ein Zelltod induziert wird, und vorzugsweise Bestimmen, ob in den nicht-krebsartigen B-Zell-Lymphozyten der genannten kultivierten B-Zell-Lymphozyten kein Zelltod induziert wird.

6. Zusammensetzung, die einen oder mehrere individualisierte CRISPR/Cas-Komplexe umfasst, die gemäß dem Verfahren eines der Ansprüche 1 bis 4 hergestellt wurden.

7. Individualisierter CRISPR/Cas-Komplex, umfassend eine Leit-RNA und eine Cas-Endonuklease,
wobei die Leit-RNA eine crRNA gemäß SEQ ID NO 37 und eine tracrRNA umfasst,
wobei die Leit-RNA eine crRNA gemäß SEQ ID NO 38 und eine tracrRNA umfasst, oder
wobei die Leit-RNA eine crRNA gemäß SEQ ID NO 39 und eine tracrRNA umfasst.

8. Ein individualisierter CRISPR/Cas-Komplex, hergestellt nach dem Verfahren gemäß einem der Ansprüche 1 bis 4, eine Zusammensetzung gemäß Anspruch 6 oder ein CRISPR/Cas-Komplex gemäß Anspruch 7, zur Verwendung bei der Behandlung von B-Zell-Lymphomen.

9. Ein individualisierter CRISPR/Cas-Komplex, hergestellt nach dem Verfahren eines der Ansprüche 1 bis 4, eine Zusammensetzung gemäß Anspruch 6 oder ein CRISPR/Cas-Komplex gemäß Anspruch 7, zur Verwendung bei der Induktion des Zelltods in krebsartigen oder präkanzerösen B-Zell-Lymphozyten.

## Revendications

1. Procédé de préparation de complexes CRISPR/Cas individualisés, comprenant les étapes consistant à
a) identifier, dans un échantillon tumoral d'un patient humain atteint d'un cancer, l'une au moins des mutations spécifiques au cancer suivantes : mutation en position 127736999 sur le chromosome 8, mutation en position 37020625 sur le chromosome 9 et mutation en position 36840626 sur le chromosome 9,
b) préparer, pour ladite au moins une mutation identifiée en a), de préférence pour deux ou toutes les mutations identifiées en a), un complexe CRISPR/Cas individualisé, chaque complexe CRISPR/Cas individualisé comprenant un ARN guide et une endonucléase Cas,
dans lequel
l'ARN guide pour le complexe CRISPR/Cas individualisé destiné à la mutation en position 127736999 sur le chromosome 8 comprend un ARNcr selon la SEQ ID NO 37 et un ARNtracr,
l'ARN guide pour le complexe CRISPR/Cas individualisé destiné à la mutation en position 37020625 sur le chromosome 9 comprend un ARNcr selon la SEQ ID NO 38 et un ARNtracr, et
l'ARN guide pour le complexe CRISPR/Cas individualisé destiné à la mutation en position 36840626 sur le chromosome 9 comprend un ARNcr selon la SEQ ID NO 39 et un ARNtracr,
l'échantillon tumoral provient de lymphocytes B.

2. Procédé selon la revendication 1,
dans lequel deux ou toutes les mutations selon a) sont identifiées, et deux ou tous les complexes CRISPR/Cas individualisés selon b) sont préparés.

3. Procédé selon la revendication 1 ou 2,
dans lequel chaque ARN guide est dirigé contre la séquence d'acide nucléique qui est spécifique pour la mutation dans des cellules cancéreuses ou précancéreuses.

4. Procédé selon l'une des revendications précédentes,
dans lequel l'endonucléase Cas est une endonucléase de type I, de type II ou de type III, de préférence une endonucléase de type I, et de manière particulièrement préférée une endonucléase CRISPR/Cas9.

5. Procédé *in vitro* pour induire la mort cellulaire dans des cellules cancéreuses ou précancéreuses de lymphocytes B, comprenant les étapes consistant à :
a) isoler les lymphocytes B d'un ou plusieurs échantillons prélevés sur un patient humain atteint d'un cancer ou sur un sujet humain supposé avoir ou développer un cancer, ce qui permet d'obtenir des lymphocytes B non cancéreux et des lymphocytes B cancéreux ou précancéreux ;
b) cultiver, de préférence *in vitro,* les lymphocytes B provenant du ou des échantillons prélevés en a) ;
c) identifier, dans les lymphocytes B cancéreux ou précancéreux, au moins l'une des mutations spécifiques au cancer suivantes : mutation en position 127736999 sur le chromosome 8, mutation en position 37020625 sur le chromosome 9 et mutation en position 36840626 sur le chromosome 9,
d) préparer un complexe CRISPR/Cas individualisé pour chacune des mutations spécifiques au cancer identifiées en c) selon l'étape b) d'un procédé de préparation de complexes CRISPR/Cas individualisés selon l'une au moins des revendications 1 à 4,
e) ajouter le ou les complexes CRISPR/Cas individualisés préparés aux lymphocytes B cultivés obtenus à l'étape b), et
f) en option, déterminer si une mort cellulaire est induite dans les lymphocytes B cancéreux ou précancéreux parmi lesdits lymphocytes B cultivés et, de préférence, déterminer si aucune mort cellulaire n'est induite dans les lymphocytes B non cancéreux parmi lesdits lymphocytes B cultivés.

6. Composition comprenant un ou plusieurs complexes CRISPR/Cas individualisés préparés par le procédé selon l'une des revendications 1 à 4.

7. Complexe CRISPR/Cas individualisé comprenant un ARN guide et une endonucléase Cas,
dans lequel
l'ARN guide comprend un ARNcr selon la SEQ ID NO 37 et un ARNtracr,
l'ARN guide comprend un ARNcr selon la SEQ ID NO 38 et un ARNtracr, ou
l'ARN guide comprend un ARNcr selon la SEQ ID NO 39 et un ARNtracr.

8. Complexe CRISPR/Cas individualisé préparé par le procédé selon l'une des revendications 1 à 4, composition selon la revendication 6, ou complexe CRISPR/Cas selon la revendication 7, destiné(e) à être utilisé(e) dans le traitement d'un lymphome à cellules B.

9. Complexe CRISPR/Cas individualisé préparé par le procédé selon l'une des revendications 1 à 4, composition selon la revendication 6, ou complexe CRISPR/Cas selon la revendication 7, destiné(e) à être utilisé(e) pour induire la mort cellulaire dans des lymphocytes B cancéreux ou précancéreux.
